# EUROPEAN PATENT APPLICATION

(11) **EP 2 050 752 A1**
(43) Date of publication of application: **22.04.2009**
(21) Application number: 07791474.5
(22) Date of filing: 27.07.2007
(51) Int. Cl.: C07D 491/18, C07C 269/00, C07C 269/06, C07C 271/24, C07D 453/06

(54) **ISOQUINUCLIDINE DERIVATIVE AND METHOD FOR PRODUCING 1-CYCLOHEXENE-1-CARBOXYLIC ACID DERIVATIVE BY USING THE SAME**

(30) Priority: 28.07.2006 JP 2006206987; 28.09.2006 JP 2006263743
(71) Applicant: The University of Tokyo, Bunkyo-ku, Tokyo 113-8654 (JP)
(72) Inventor: FUKUYAMA, Tohru, Tokyo 113-8654 (JP); YOKOSHIMA, Satoshi, Tokyo 113-8654 (JP); SATOH, Nobuhiro, Tokyo 113-8654 (JP)
(74) Representative: Kuhnen & Wacker
(86) International application number: PCT/JP2007/064780
(87) International publication number: WO 2008/013269

(57) **Abstract**

The present invention provides an isoquinuclidine derivative which can be used to easily synthesize oseltamivir or an analog thereof. In particular, the present invention provides an isoquinuclidine derivative represented by the following formula (1) or an enantiomer thereof: 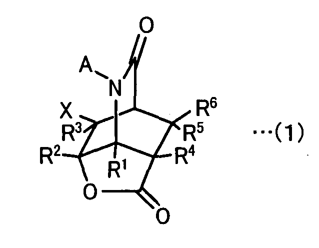 wherein in the formula (1), A represents a protective group for the nitrogen atom; R¹ to R¹ each independently represent an alkyl group which may have a substituent, an aryl group which may have a substituent, or a hydrogen atom; and X represents a halogen atom.

## Description

### Technical Field

The present invention relates to an isoquinuclidine derivative and a method for manufacturing a 1-cyclohexene-1-carboxylic acid derivative.

### Background Art

Heretofore, a 1-cyclohexene-1-carboxylic acid derivative, in particular, oseltamivir ((3R,4R,5S)-4-acetylamino-5-amino-3-(1-ethylpropoxy)-1-cyclohexene-1-carboxylic acid ethyl ester) and its salt are strong inhibitors against virus neuraminidases and have drawn attention as a miracle drug for influenza. As disclosed, for example, in Patent Document 1, this oseltamivir is synthesized through a multi-stage process using (-)-quininic acid or (-)-shikimic acid as a starting material. In addition, methods for synthesizing oseltamivir using a starting material different from that described above have been disclosed in Non-Patent Documents 1 and 2.
[Patent Document 1] International Publication WO 98/07685
[Non-Patent Document 1] J. Am. Chem. Soc., 2006, vol. 128, pp. 6310 to 6311
[Non-Patent Document 2] J. Am. Chem. Soc., 2006, vol. 128, pp. 6312 to 6313

### Disclosure of Invention

However, according to the method for synthesizing oseltamivir disclosed in the Patent Document 1, since the starting material is a natural product which is not easily available, it cannot be said that the above method is suitably applied to mass production. In addition, the synthesis methods disclosed in Non-Patent Documents 1 and 2 are superior to the method disclosed in the Patent Document 1 since a natural product is not used as the starting material; however, since a very expensive reagent and/or a reagent which cannot be easily handled is used, it cannot be always said that the methods are suitably applied to mass production. In addition, in order to develop a safer and more active medicine, it is important to synthesize a novel 1-cyclohexene-1-carboxylic acid derivative to be used instead of oseltamivir; however, it is necessary to develop the environment in which a synthetic intermediate suitably used to obtain a novel medicine as described above is provided at a reasonable cost.

The present invention has been conceived in consideration of the situation described above, and one object of the present invention is to provide an isoquinuclidine derivative which can be easily synthesized into a 1-cyclohexene-1-carboxylic acid derivative. In addition, another object of the present invention is to provide a novel method for manufacturing a 1-cyclohexene-1-carboxylic acid derivative.

Through intensive research on a synthetic route using a reverse synthesis method of oseltamivir carried out in order to achieve at least one of the above objects, the inventor of the present invention assumed that a certain type of isoquinuclidine derivative was useful to synthesize oseltamivir or an analog thereof, and after the above isoquinuclidine derivative was actually synthesized, it was confirmed that oseltamivir could be obtained therefrom; hence, the present invention was made.

An isoquinuclidine derivative of the present invention is a compound represented by formula (1) or an enantiomer thereof, a compound represented by formula (2) or an enantiomer thereof, or a compound represented by formula (3) or an enantiomer thereof. The isoquinuclidine derivative may be used as a synthetic intermediate for manufacturing a 1-cyclohexene-1-carboxylic acid derivative, such as oseltamivir. Since pyridine, which is available at a reasonable cost, may be used as a starting material, the isoquinuclidine derivative is suitable for mass production.

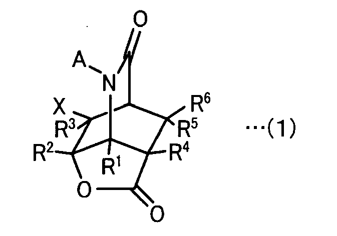

In the formula (1), A represents a protective group for a nitrogen atom; R¹ to R⁶ each independently represent an alkyl group which may have a substituent, an aryl group which may have a substituent, or a hydrogen atom; and X represents a halogen atom.

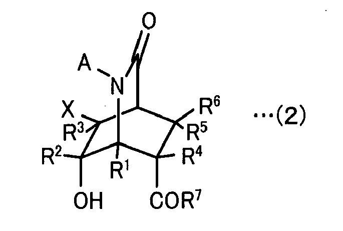

In the formula (2), A represents a protective group for a nitrogen atom; R¹ to R⁶ each independently represent an alkyl group which may have a substituent, an aryl group which may have a substituent, or a hydrogen atom; R⁷ represents a hydroxyl group, an amino group which may have a substituent, a hydrazino group which may have a substituent, an alkoxy group which may have a substituent, or a hydroxyamino group which may have a substituent; and X represents a halogen atom.

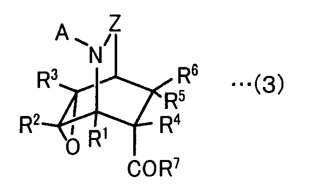

In the formula (3), A represents a protective group for a nitrogen atom; R¹ to R⁶ each independently represent an alkyl group which may have a substituent, an aryl group which may have a substituent, or a hydrogen atom; R⁷ represents a hydroxyl group, an amino group which may have a substituent, a hydrazino group which may have a substituent, an alkoxy group which may have a substituent, or a hydroxyamino group which may have a substituent; and Z represents a -CH₂- group or a -C(=O)- group.

In the present invention, unless otherwise specifically stated, when the structure is represented by "formula (1)", the formula (1) also represents enantiomer of the structure represented thereby. The same thing can also be said for formulas (2), (3), and the like.

A method for manufacturing a 1-cyclohexene-1-carboxylic acid derivative according to one aspect of the present invention including: a step (a1) of, after the -OH group of the isoquinuclidine derivative represented by the formula (2) is protected or is converted into an elimination group, converting the -COR⁷ group into a non-substituted or a substituted amino group by a rearrangement reaction; and a step (b1) of disconnecting the bond between a nitrogen atom and a carbonyl carbon forming a lactam of an isoquinuclidine derivative obtained in the step (a1) and eliminating a hydrogen atom at the α position and X at the β position of the carbonyl carbon forming the lactam to form an unsaturated bond.

A method for manufacturing a 1-cyclohexene-1-carboxylic acid derivative according to another aspect of the present invention including: a step of disconnecting the bond between the nitrogen atom and the carbonyl carbon forming the lactam of the isoquinuclidine derivative represented by the formula (2) and eliminating the hydrogen atom at the αβ position and X at the β position of the carbonyl carbon forming the lactam to form an unsaturated bond. In this method, the bond between the nitrogen atom and the carbonyl carbon forming the lactam of the isoquinuclidine derivative may be disconnected after the -OH group of the isoquinuclidine derivative represented by the formula (2) is protected or is converted into an elimination group.

A method for manufacturing a 1-cyclohexene-1-carboxylic acid derivative according to another aspect of the present invention including: a step (a2) of converting the -COR⁷ group of the isoquinuclidine derivative (where Z represents a -C(=O)-group) represented by the formula (3) into a non-substituted or a substituted amino group by a rearrangement reaction; and a step (b2) of disconnecting the bond between a nitrogen atom and a carbonyl carbon forming a lactam of an isoquinuclidine derivative obtained in the step (a2) and forming an unsaturated bond between the α position and the β position of the carbonyl carbon forming the lactam concomitant with opening of an epoxy ring.

A method for manufacturing a 1-cyclohexene-1-carboxylic acid derivative according to another aspect of the present invention including: a step of disconnecting the bond between the nitrogen atom and the carbonyl carbon forming the lactam of the isoquinuclidine derivative (where Z represents a -C(=O)- group) represented by the formula (3) and forming an unsaturated bond between the α position and the β position of the carbonyl carbon forming the lactam concomitant with opening of the epoxy ring.

### Best Modes for Carrying Out the Invention

In the isoquinuclidine derivatives of the present invention represented by the formulae (1) to (3), A may function as a protective group for the nitrogen atom, and for example, an alkoxycarbonyl group may be mentioned. As particular examples of the alkoxycarbonyl group, a benzyloxycarbonyl (Cbz) group, a tert-butoxycarbonyl (Boc) group, a 8,9-fluorenylmethoxycarbonyl (Fmox) group, a t-amyloxycarbonyl (Aoc) group, a 4-methoxybenzyloxycarbonyl group, a 2-chloro-benzyloxycarbonyl group, an adamantyloxycarbonyl group, a methoxycarbonyl group, an ethoxycarbonyl group, an isopropoxycarbonyl group, an allyloxycarbonyl (Alloc) group, a 2,2,2-trichloroethoxycarbonyl group, a phenyloxycarbonyl group, and the like may be mentioned. In addition, as protective groups other than the alkoxycarbonyl group, for example, a benzene sulfonyl group, a methane sulfonyl group, an acetyl group, a benzoyl group, a formyl group, and the groups described above including substituents may be mentioned.

In the isoquinuclidine derivatives of the present invention represented by the formulas (1) to (3), as non-substituted alkyl groups which may be used as R¹ to R⁶, for example, there may be mentioned chain alkyl groups, such as a methyl group, an ethyl group, a n-propyl group, an isopropyl group, a n-butyl group, an isobutyl group, a sec-butyl group, a pentyl group, an isopentyl group, a neopnentyl group, a 1-ethylpropyl group, a hexyl group, an isohexyl group, a 2,2-dimethylbutyl group, and a 3,3-dimethylbutyl group; and cyclic alkyl groups, such as a cyclopentyl group, a cyclohexyl group, and a cycloheptyl group. In addition, as substituted alkyl groups which may be used as R¹ to R⁶, for example, there may be mentioned groups each having an alkyl group, a cycloalkyl group, an aryl group, an alkoxy group, or the like on at least one carbon atom of a non-substituted alkyl group. In this embodiment, as the alkyl and the cycloalkyl groups, those mentioned above by way of example may be used; as the aryl group, for example, a phenyl group, a naphthyl group, an anthryl group, and a phenanthryl group may be mentioned; and as the alkoxy group, for example, a methoxy group and an ethoxy group may be mentioned. As non-substituted aryl groups which may be used as R¹ to R⁶, for example, a phenyl group, a naphthyl group, an anthryl group, and a phenanthryl group may be mentioned; and as substituted aryl groups, there may be mentioned groups each having an alkyl group, a cycloalkyl group, an aryl group, an alkoxy group, or the like on at least one carbon atom of a non-substituted aryl group. As the substituents, the alkyl group, the cycloalkyl group, the aryl group, and the alkoxy group, which are mentioned above by way of example, may be used.

In the isoquinuclidine derivatives of the present invention represented by the formula (2) or (3), as a substituted amino group which may be used as R⁷, for example, there may be mentioned a hydroxyamino group (HO-NH-) or a substituted hydroxyamino group (RO-NH-, R represents an alkyl group) as well as a primary amino group, such as a methylamino group, an ethylamino group, or a phenylamino group, or a secondary amino group, such as a dimethylamino group, a diethylamino group, or a diphenylamino group. As a substituted hydrazino group which may be used as R⁷, for example, a monomethylhydrazino group or a dimethylhydrazino group may be mentioned.

In the isoquinuclidine derivatives of the present invention represented by the formula (1) or (2), although a halogen atom which may be used as X is not particularly limited, for example, chlorine, bromine, or iodine may be mentioned. Among those halogen atoms, bromine is preferably mentioned.

A method for manufacturing the isoquinuclidine derivative of the present represented by the formula (1) may include, as shown in a synthesis route of the following chemical equation 4:
a step of performing the Diels-Alder reaction between a 1,2-dihydropyridine derivative represented by formula (11) and an acrolein compound represented by formula (12) to obtain an isoquinuclidine derivative having a formyl group represented by formula (13);
a step of oxidizing the formyl group to obtain an isoquinuclidine derivative having a carboxyl group represented by formula (14);
a step of performing an intramolecular lactonization reaction using the carboxyl group to obtain an isoquinuclidine derivative having a lactone represented by formula (15); and
a step of oxidizing methylene adjacent to a nitrogen atom to obtain an isoquinuclidine derivative represented by the formula (1).

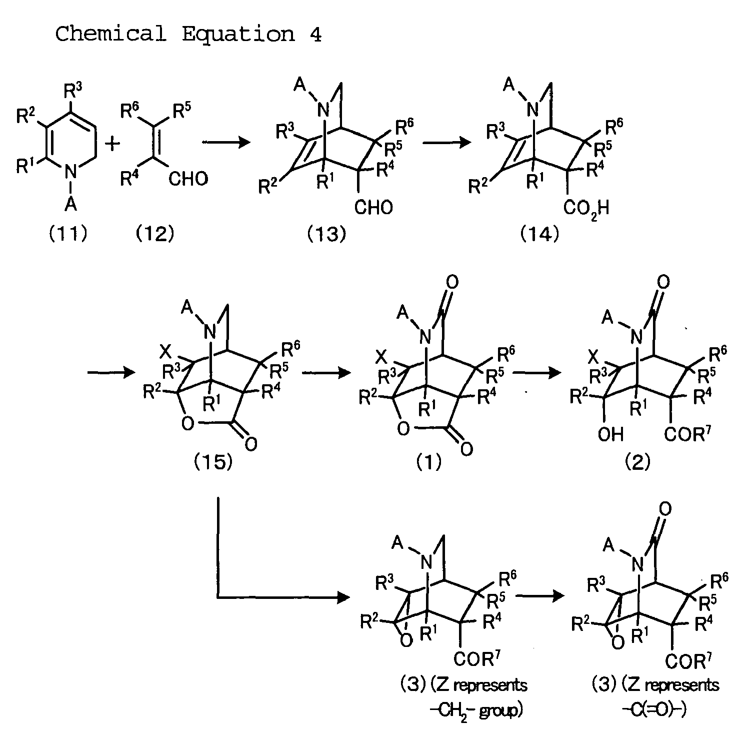

According to this manufacturing method, the 1,2-dihydropyridine derivative represented by the formula (11) may be obtained, for example, by performing a hydrogenation reaction of pyridine and a reaction for introducing a protective group A to the nitrogen atom. In addition, the isoquinuclidine derivative having a formyl group represented by the formula (13) is obtained by performing the Diels-Alder reaction between the 1,2-dihydropyridine derivative represented by the formula (11) and the acrolein compound represented by the formula (12) in the presence of, for example, a MacMillan catalyst ((5S) or (5R)-2,2,3-trimethyl-5-phenylmethyl-4-imidazolidinone hydrochloride). By this Diels-Alder reaction, an exo form is also produced as well as the endo form represented by the formula (13); however, in the step in which the isoquinuclidine derivative having a carboxyl group represented by the formula (14) is converted into the isoquinuclidine derivative having a lactone represented by the formula (15), the product (lactone) is obtained only from the endo form, and the exo form remains in the form of a carboxylic acid; hence, the end form and the exo form can be easily separated from each other by a separation operation. In addition, the MacMillan catalyst can be easily synthesized from D- or L-phenylalanine which is commercially available at a reasonable price. In this case, depending on the 5S or the 5R configuration of the MacMillan catalyst, the absolute configuration represented by the formula (13) or the absolute configuration of an enantiomer thereof is determined. Furthermore, the isoquinuclidine derivative having a carboxyl group represented by the formula (14) is obtained by oxidizing the formyl group of the isoquinuclidine derivative represented by the formula (13), for example, using an oxidizing agent, such as sodium chlorite. The isoquinuclidine derivative represented by the formula (15) is obtained by intramolecular lactonization of the isoquinuclidine derivative represented by the formula (14), for example, using an iodine lactonization reaction or a similar reaction (for example, using bromine instead of iodine). The isoquinuclidine derivative represented by the formula (15) can be converted into the isoquinuclidine derivative represented by the formula (1) by oxidation, for example, using ruthenium oxide (IV) or the like. Since ruthenium oxide (IV) can be recycled, an oxidation reaction using this compound is suitable for industrialization.

In addition, as a method for manufacturing the isoquinuclidine derivative of the present invention represented by the formula (2), for example, as shown in the chemical equation 4, a method may be used in which a nucleophilic agent is allowed to reach with the carbonyl carbon of the lactone of the isoquinuclidine derivative represented by the formula (1) to form the isoquinuclidine derivative represented by the formula (2). Furthermore, as a method for manufacturing the isoquinuclidine derivative of the present invention represented by the formula (3), for example, as shown in the chemical equation 4, a method may be used in which a nucleophilic agent is allowed to reach with the carbonyl carbon of the lactone of the isoquinuclidine derivative represented by the formula (15) to form the isoquinuclidine derivative having an epoxy group represented by the formula (3) (Z represents a -CH₂- group), followed by oxidation using ruthenium oxide to form the isoquinuclidine derivative represented by the formula (3) (Z represents a -C(=O)-group).

A method for manufacturing a 1-cyclohexene-1-carboxylic acid derivative of the present invention may include:
a step (a1) of, after the -OH group of the isoquinuclidine derivative represented by the formula (2) is protected or is converted into an elimination group, converting the -COR⁷ group into a non-substituted or a substituted amino group by a rearrangement reaction; and
a step (b1) of disconnecting the bond between a nitrogen atom and a carbonyl carbon forming a lactam of an isoquinuclidine derivative obtained in the above step (a1) and eliminating a hydrogen atom at the α position and X at the β position of the carbonyl carbon forming the lactam to form an unsaturated bond.

In this method, when the -OH group is protected by a protective group in the step (a1), after the unsaturated bond is formed in the step (b1), deprotection is performed to recover the -OH group, and under this condition, an aziridine is formed by the Mitsunobu reaction, or after the -OH group is converted into an elimination group, the elimination group is eliminated by the nitrogen atom forming the lactam to form an aziridine. Subsequently, the aziridine ring may be opened by an alcohol or an alkoxide to introduce an alkoxy group to the 3-position of 1-cyclohexene. On the other hand, when the -OH group is converted into an elimination group in the step (a1), at the same time when the unsaturated bond is formed in the step (b1) or thereafter, the elimination group is eliminated by the nitrogen atom forming the lactam to form an aziridine, and subsequently, the aziridine ring may be opened by an alcohol or an alkoxide to introduce an alkoxy group to the 3-position of 1-cyclohexene. In addition, the isoquinuclidine derivative represented by the formula (2) may be obtained by disconnecting the bond between the oxygen atom and the carbonyl carbon forming the lactone of the isoquinuclidine derivative represented by the formula (1).

For example, in a synthesis route shown in the following chemical equation 5, after the bond between the oxygen atom and the carbonyl carbon forming the lactone of the isoquinuclidine derivative represented by the formula (1) is disconnected by ammonia, hydrazine, water, or an alcohol to form an isoquinuclidine derivative (see the formula (2)) having an aminocarbonyl group, a hydrazinocarbonyl group, a carboxyl group, or an alkoxycarbonyl group, respectively, the OH group is protected by a protective group (such as an acetyl group, a benzoyl group, or a silyl group (such as a trimethylsilyl group, a triethylsilyl group, or a t-butyldimetylsilyl group) or is converted into an elimination group (such as a mesyloxy group or a tosyloxy group). Subsequently, as for the compound having an aminocarbonyl group, the aminocarbonyl group is converted into an alkoxycarbonylamino group by the Hofmann rearrangement in the presence of an alcohol, and as for the compounds having a hydrazinocarbonyl group, a carboxyl group, and an alkoxycarbonyl group, the groups are each once converted into a -CON₃ group and are then each converted into an alkoxycarbonylamino group by the Curtius rearrangement in the presence of an alcohol. However, after the rearrangements described above are performed without using an alcohol, the above groups may be converted into non-substituted amino groups by decarbonization. Subsequently, when the bond between the nitrogen atom and the carbonyl carbon forming the lactam is disconnected by a nucleophilic agent, such as sodium ethoxide, under basic conditions, the elimination of HX is advanced concomitant with this disconnection, and a 1-cyclohexene-1-carboxylic acid derivative is obtained. On the other hand, when the disconnection is performed using an alcohol under acidic conditions, a process is performed using a basic material after the disconnection to advance the elimination of HX, so that a 1-cyclohexene-1-carboxylic acid derivative can be obtained. The 1-cyclohexene-1-carboxylic acid ester thus obtained can be easily converted into oseltamivir or an analog thereof.

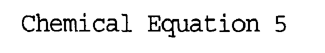

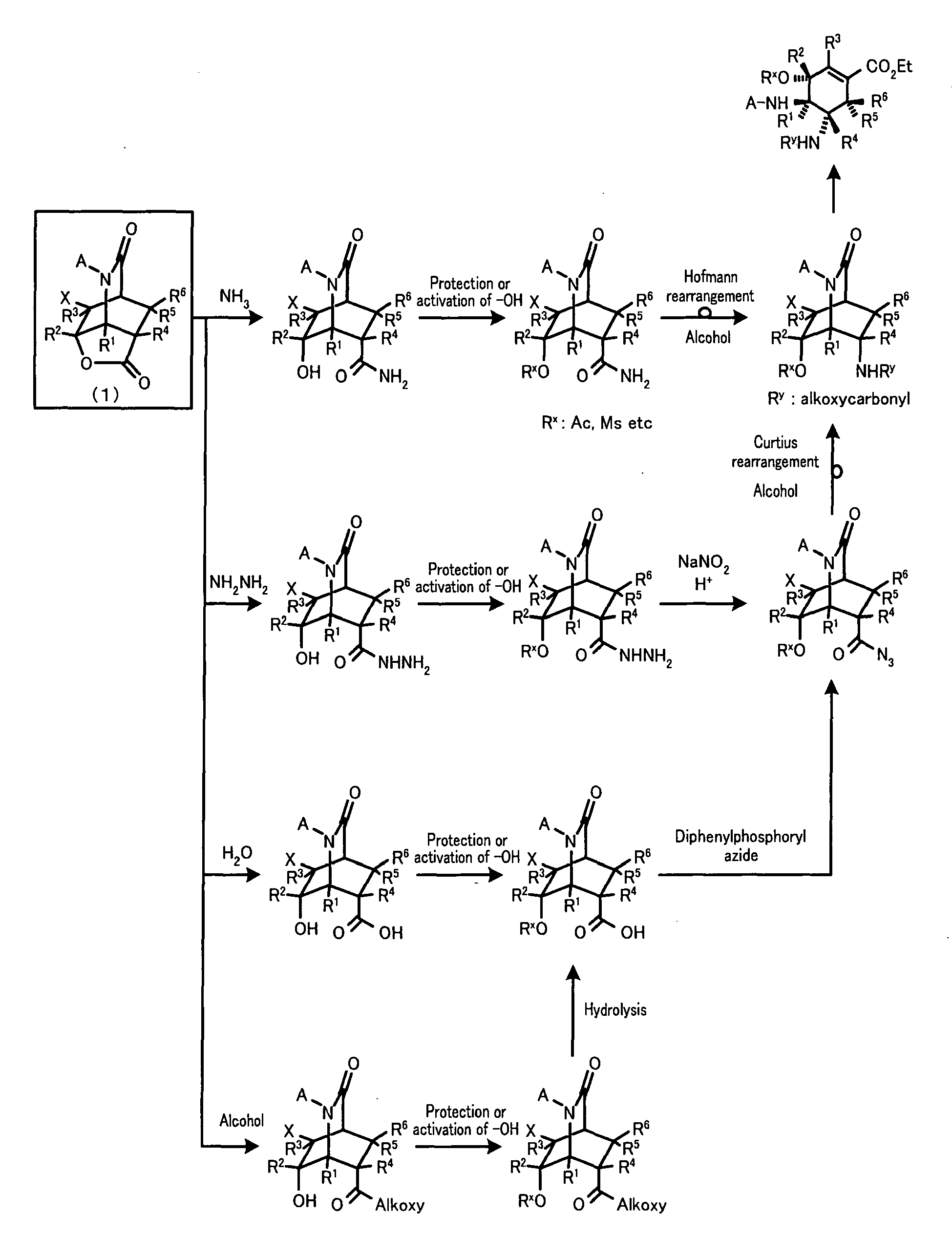

Alternatively, a method for manufacturing the 1-cyclohexene-1-carboxylic acid derivative of the present invention may include a step in which the bond between the nitrogen atom and the carbonyl carbon forming the lactam of the isoquinuclidine derivative represented by the formula (2) is disconnected, and the hydrogen atom at the α position of the carbonyl carbon and X at the β position thereof are eliminated to form an unsaturated bond.

In the above step, without protecting the -OH group of the isoquinuclidine derivative represented by the formula (2) or converting it into another group, the bond between the nitrogen atom and the carbonyl carbon forming the lactam of the above isoquinuclidine derivative may be disconnected; however, after the -OH group of the isoquinuclidine derivative represented by the formula (2) is protected or is converted into an elimination group, the bond between the nitrogen atom and the carbonyl carbon forming the lactam of the above isoquinuclidine derivative may be disconnected. In addition, when the -OH is protected by a protective group, after the unsaturated bond is formed in the step described above, deprotection is performed to recover the - OH group, and under this condition, an aziridine is formed by the Mitsunobu reaction, or after the -OH group is converted into an elimination group, the elimination group is eliminated by the nitrogen atom forming the lactam to form an aziridine. Subsequently, the aziridine ring may be opened by an alcohol or an alkoxide to introduce an alkoxy group to the 3-position of 1-cyclohexene. On the other hand, when the -OH group is converted into an elimination group in the step described above, at the same time when the unsaturated bond is formed in the above step or thereafter, the elimination group is eliminated by the nitrogen atom forming the lactam to form an aziridine, and subsequently, the aziridine ring may be opened by an alcohol or an alkoxide to introduce an alkoxy group to the 3-position of 1-cyclohexene. When it is desired to convert the -COR⁷ group into a non-substituted or a substituted amino group, at an optional stage after the lactam ring is opened, the Hofmann rearrangement or the Curtius rearrangement may be used. In addition, the isoquinuclidine derivative represented by the formula (2) may be obtained by disconnecting the bond between the oxygen atom and the carbonyl carbon forming the lactone of the isoquinuclidine derivative represented by the formula (1).

For example, in a synthesis route shown by the following chemical equation 6, after the bond between the oxygen atom and the carbonyl carbon forming the lactone of the isoquinuclidine derivative represented by the formula (1) is disconnected by ammonia, hydrazine, water, or an alcohol to form an isoquinuclidine derivative (see the formula (2)) having an aminocarbonyl group, a hydrazinocarbonyl group, a carboxyl group, or an alkoxycarbonyl group, respectively, the OH group is protected by a protective group (such as an acetyl group, a benzoyl group, or a silyl group (such as a trimethylsilyl group, a triethylsilyl group, or a t-butyldimetylsilyl group) or is converted into an elimination group (such as a mesyloxy group or a tosyloxy group). However, the OH group may remain in a free state. Subsequently, when the bond between the nitrogen atom and the carbonyl carbon forming the lactam is disconnected by a nucleophilic agent, such as sodium ethoxide, under basic conditions, the elimination of HX is advanced concomitant with this disconnection, and a 1-cyclohexene-1-carboxylic acid derivative is obtained. On the other hand, when the disconnection is performed using an alcohol under acidic conditions, a process is performed using a basic material after the disconnection to advance the elimination of HX, so that a 1-cyclohexene-1-carboxylic acid derivative can be obtained. At this stage, when being placed in a free state, the -OH group is protected by a protective group or is converted into an elimination group. Subsequently, as for the compound having an aminocarbonyl group, the aminocarbonyl group is converted into an alkoxycarbonylamino group by the Hofmann rearrangement in the presence of an alcohol. In addition, as for the compounds having a hydrazinocarbonyl group, a carboxyl group, and an alkoxycarbonyl group, the groups are each once converted into a - CON₃ group and are then each converted into an alkoxycarbonylamino group by the Curtius rearrangement in the presence of an alcohol. The 1-cyclohexene-1-carboxylic acid ester thus obtained can be easily converted into oseltamivir or an analog thereof. In addition, by performing the above rearrangements without using an alcohol for decarbonization, a non-substituted amino group may be obtained by the conversion.

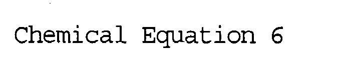

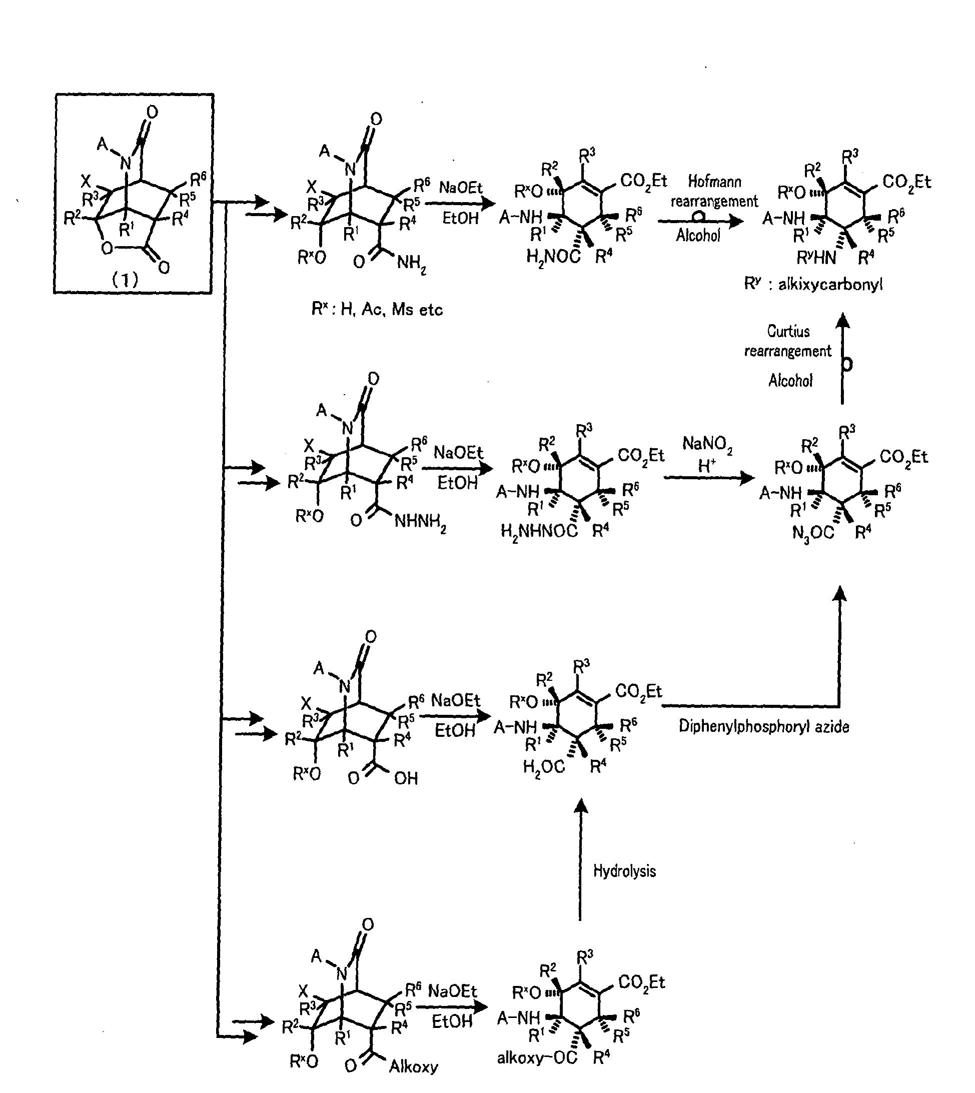

A method for manufacturing the 1-cyclohexene-1-carboxylic acid derivative of the present invention may include:
a step (a2) of converting the -COR⁷ group of the isoquinuclidine derivative (where Z represents a -C(=O)- group) represented by the formula (3) into a non-substituted or a substituted amino group by a rearrangement reaction; and
a step (b2) of disconnecting the bond between a nitrogen atom and a carbonyl carbon forming a lactam of an isoquinuclidine derivative obtained in the above step (a2) and forming an unsaturated bond between the α position and the β position of the carbonyl carbon forming the lactam concomitant with opening of an epoxy ring.

In this method, after the unsaturated bond is formed in the step (b2), an aziridine is formed by the Mitsunobu reaction, or after the -OH group is converted into an elimination group, the elimination group is eliminated by the nitrogen atom forming the lactam to form an aziridine, and subsequently, the aziridine ring may be opened by an alcohol or an alkoxide to introduce an alkoxy group to the 3-position of 1-cyclohexene.

For example, in a synthesis route shown in the upper column of the following chemical equation 7, the -CO₂H- group of the isoquinuclidine derivative represented by the formula (3) is converted into a -CON₃- group, and this is converted into an alkoxycarbonylamino group, such as allyloxycarbonylamino group, by the Curtius rearrangement in the presence of an alcohol. Next, when the bond between the nitrogen atom and the carbonyl carbon forming the lactam is disconnected by a nucleophilic agent, such as sodium ethoxide, under basic conditions, as the disconnection is advanced, an unsaturated bond is formed between the α position and the β position of the carbonyl carbon forming the lactam concomitant with opening of the epoxy ring, so that a 1-cyclohexene-1-carboxylic acid derivative can be obtained. On the other hand, when the disconnection is performed using an alcohol under acidic conditions, a process is performed using a basic material after the disconnection to open the epoxy ring and to form the unsaturated bond, so that a 1-cyclohexene-1-carboxylic acid derivative can be obtained. The 1-cyclohexene-1-carboxylic acid ester thus obtained can be easily converted into oseltamivir or an analog thereof.

Alternatively, a method for manufacturing the 1-cyclohexene-1-carboxylic acid derivative of the present invention may include a step of disconnecting the bond between the nitrogen atom and the carbonyl carbon forming the lactam of the isoquinuclidine derivative (where Z represents a -C(=O)- group) represented by the formula (3) and forming an unsaturated bond between the α position and the β position of the carbonyl carbon forming the lactam concomitant with opening of the epoxy-ring.

In this method, after the unsaturated bond is formed in the step described above, the -COR⁷ group may be converted into a non-substituted or a substituted amino group by a rearrangement reaction. Subsequently, after an aziridine is formed by the Mitsunobu reaction, or after an aziridine is formed such that the -OH group is converted into an elimination group, and the elimination group is then eliminated by the nitrogen atom forming the lactam, the aziridine ring may be opened using an alcohol or an alkoxide so as to introduce an alkoxy group to the 3-position of 1-cyclohexene.

For example, in a synthesis route shown in the lower column of the following the chemical equation 7, after the bond between the nitrogen atom and the carbonyl carbon forming the lactam is first disconnected, the carboxyl group is converted into the amino group by the Curtius rearrangement.

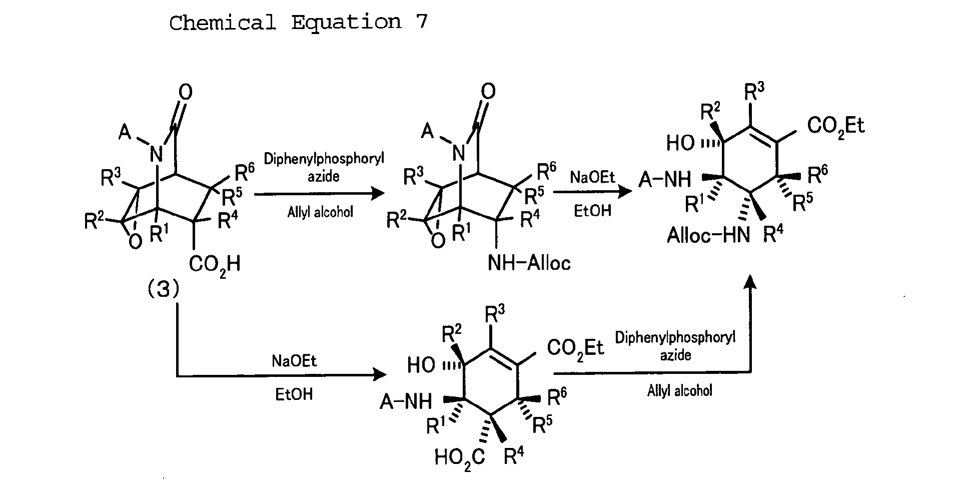

### Example 1

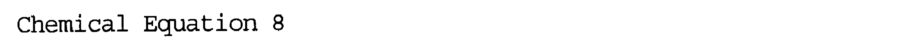

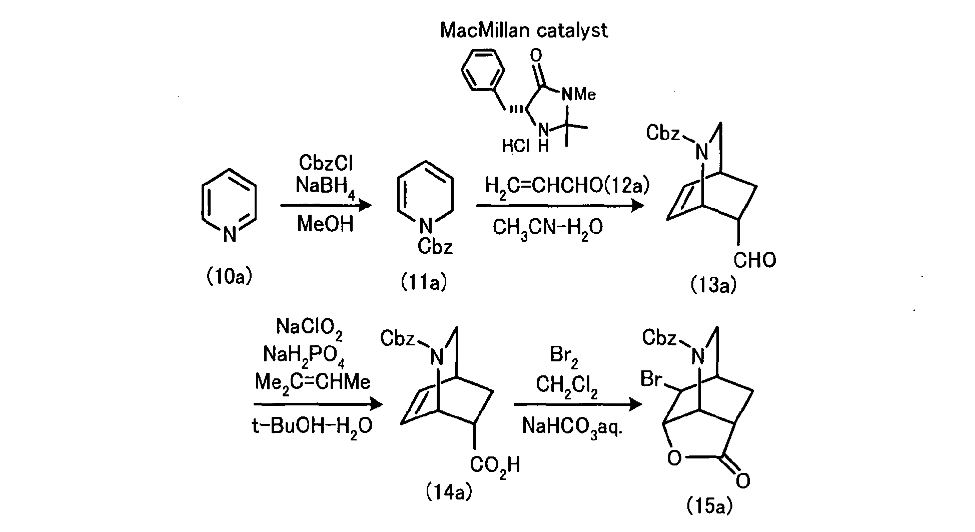

With reference to the chemical equation 8, a particular experimental procedure will be described. In an argon atmosphere, after pyridine (10a) (12.1 ml, 150 mmol) was dissolved in methanol (500 ml) and was then cooled to -78°C, sodium boron hydride (6.79 g, 180 mmol) was added. Benzyl chloroformate (21.4 ml, 150 mmol) was dripped for 12 minutes. During the dripping, a white precipitate was generated. After the dripping was finished, and stirring was then performed at -78°C for 12 minutes, the temperature of this reaction liquid was increased to 0°C, and hydrochloric acid (1 M aqueous solution, 500 ml) was added, thereby stopping the reaction. The volume of the reaction liquid was decreased to one third by condensation under reduced pressure and was then processed by an ethyl acetate-1 M hydrochloric acid solution for separation. After an aqueous layer was extracted twice with ethyl acetate, a collected organic layer was sequentially washed with saturated sodium bicarbonate water and saturated aqueous sodium chloride. After the organic layer was dried using anhydrous sodium sulfate, condensation was performed under reduced pressure, so that 31 g of dihydropyridine (11a) in the form of a pale yellow oily material was obtained.

The dihydropyridine (11a) thus obtained was used for the next reaction without being purified. That is, the dihydropyridine (11a)(31g, 144 mmol) was dissolved in an acetonitrile-water mixed solvent (95: 5, 370 ml), acrolein (12a) (30.0 ml, 449 mmol) and a MacMillan catalyst (5R configuration, 3.82 g, 15.0 mmol) derived from D-phenylalanine were sequentially added in an argon atmosphere, an acetonitrile-water mixed solvent (95: 5, 80 ml) was further added, and stirring was performed at room temperature for approximately 10 hours, so that the Diels-Alder reaction was performed. After this reaction liquid was diluted with diethyl ether and water and was then transferred to a separating funnel, an aqueous layer was separated and was then extracted with diethyl ether. A collected organic layer was sequentially washed with water and saturated aqueous sodium chloride. After the organic layer was dried using anhydrous sodium sulfate, condensation was performed under reduced pressure, so that an isoquinuclidine derivative (13a) having a formyl group in the form of a yellow oily material was obtained. After this oily material was dissolved in a t-butanol-water mixed solvent (3: 1, 300 ml), 2-methyl-2-butene (80 ml, 748 mmol) and sodium dihydrogenphosphate (34.9 g, 224 mmol) were added while the reaction liquid was cooled using an ice bath, and sodium chlorite (33.8 g, 374 mmol) was dividedly added 5 times. After stirring was performed for 10 minutes under ice cooling conditions, the ice bath was removed, and stirring was performed at room temperature for 50 minutes. Subsequently, this reaction liquid was again cooled using an ice bath, and sodium thiosulfate was added to the reaction liquid, so that the reaction was stopped. The reaction liquid was separated using an ethyl acetate-1 M hydrochloric acid solution, and an aqueous layer was extracted with ethyl acetate. A collected organic layer was washed with saturated aqueous sodium chloride and was then condensed under reduced pressure so as to decrease the volume to approximately one fifth. The condensed organic layer was sufficiently extracted with saturated sodium bicarbonate water, so that an aqueous solution containing an isoquinuclidine derivative (14a) having a carboxyl group was obtained. This aqueous solution was used for the next reaction without being further processed. That is, methylene chloride (approximately 300 ml) was added to this aqueous solution, and while stirring was vigorously performed at room temperature, iodine was dripped until the color thereof did not disappear. Subsequently, sodium sulfite was added, so that the reaction was stopped. The reaction liquid was extracted 3 times with ethyl acetate, and a collected organic layer was washed with water and saturated aqueous sodium chloride. The organic layer was dried by anhydrous sodium sulfate and was then condensed under reduced pressure, so that a gummy material was obtained. After celite filtration was performed, and purification was then performed using column chromatography (33% of ethyl acetate-hexane), a purified product was saturated in an ethyl acetate-hexane solution at 70°C and was then cooled to room temperature, and subsequently, a small amount of seed was added, so that a crystalline product was obtained. The crystal product thus obtained was washed ethyl acetate which was cooled with ice, so that an isoquinuclidine derivative (15a) (3.64 g, Y. 6.6%) having a lactone was obtained in the form of a pale yellow crystal. The spectrum data of this isoquinuclidine derivative (15a) is as follows.

¹H NMR(CDCl₃): δ(ppm)7.38(s,5H), 5.20(m,2H), 5.01(t, J=5.2 Hz, (2/3)1H), 4.91(t, J=5.2 Hz, (2/3)1H), 4.86(m, (1/3)1H), 4.85(m, (1/3)1H), 4.28(d, J=11.2 Hz, 1H), 4.05(d, J=11.2 Hz, 1H), 3.33(d, J=11.2 Hz, 1H), 2.86(m, 1H), 2.50(br s, (1/3)1H}, 2.44(br s, (2/3)1H), 2.26(dd, J=13.5, 11.2 Hz, 1H), 2.03(d, J=13.5 Hz, 1H). The compound thus obtained was observed as a mixture (approximately 2: 1) of rotational isomers derived from the Cbz group.

### Example 2

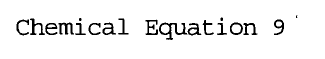

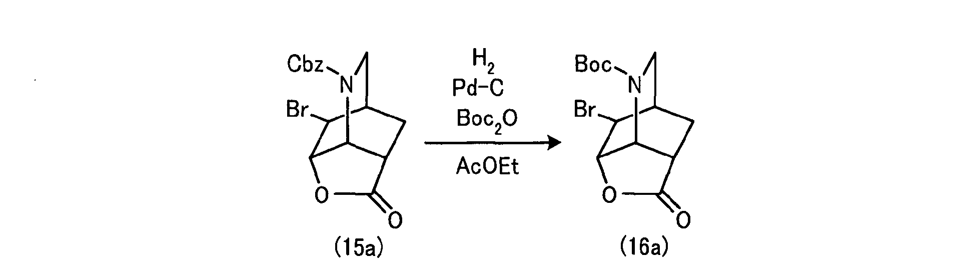

As shown in the chemical equation 9, the isoquinuclidine derivative (15a) (3.91 g, 10.7 mmol) was dispersed in ethyl acetate (11 ml), and after di-t-butyl dicarbonate (7.01 g, 32.1 mmol) and palladium-carbon (wet type, 10%, 2.39 g) were added in an argon atmosphere, stirring was vigorously performed in a hydrogen atmosphere for approximately 11 hours. After the palladium-carbon was removed by celite filtration, the filtrate was condensed. The residue was purified by silica gel column chromatography (25%-30% of ethyl acetate-hexane). After an obtained oily material was dissolved in a small amount of ethyl acetate and was then concussed for a while to obtain a crystalline product, this product was diluted with hexane and was then filtered, so that an isoquinuclidine derivative (16a) in which the protective group was changed from the Cbz group to the Boc group was obtained in the form of a white crystal (3.11 g, Y. 87.4%). The spectrum data of this isoquinuclidine derivative (16a) is as follows.

¹H NMR (CDCl₃):δ(ppm) 4.95 (t, J = 5.3 Hz, (2/3)1H), 4.89 (d, J = 5.3 Hz, (2/3)1H), 4.86 (d, J = 5.1 Hz, (1/3)1H), 4.76 (t, J = 5.1 Hz, (1/3)1H), 4.26 (d, J = 3.6 Hz, 1H), 3.94 (d, J = 11.2 Hz, 1H), 3.25 (d, J = 11.2 Hz, 1H), 2.84 (m, 1H), 2.48 (br s, (1/3)1H), 2.42 (br s, (2/3)1H), 2.26 (dd, J = 14.4, 10.8 Hz, 1H), 2.01 (d, 14.4 Hz, 1H), 1.50 (s, 9H). The compound thus obtained was observed as a mixture (approximately 2: 1) of rotational isomers derived from the Boc group.

In addition, the isoquinuclidine derivative (16a) in which the protective group for the nitrogen atom was converted into a t-Bu group may also be obtained in a way such that after phenyl chloroformate was allowed to react with pyridine (10a) to form N-phenyloxycarbonyl dihydropyridine, an isoquinuclidine derivative (having a phenyloxycarbonyl group instead of the Cbz group of the compound (15a)) having a lactone was synthesized by the Diels-Alder reaction, oxidation reaction, and lactonization reaction in accordance with those performed in Example 1 and was then allowed to react with KOt-Bu.

### Example 3

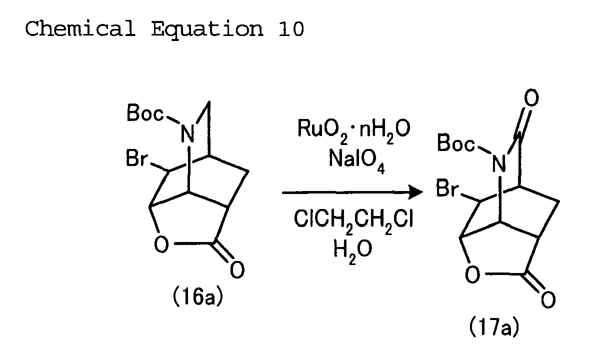

As shown in the chemical equation 10, after 1,2-dichloroethane (24.0 ml) and water (12.0 ml) were added to the isoquinuclidine derivative (16a) (1.56 g, 4.69 mmol) in an argon atmosphere, ruthenium dioxide n-hydrate (116 mg, 0.469 mmol) and sodium periodate (3.01 g, 14.07 mmol) were added, and this mixture was heated to 80°C and was stirred for 1 hour and 40 minutes. In addition, after sodium periodate (1.36 g, 6.36 mmol) was further added, and stirring was further performed for 1 hour and 30 minutes at 80°C, cooling was performed to room temperature, and 30 drops of 2-propanol was added, so that the reaction was stopped. After this reaction liquid was diluted with ethyl acetate, and a precipitate was processed by celite filtration, the filtrate was transferred to a separating funnel and was then sequentially washed with a saturated sodium hydrogen carbonate aqueous solution and saturated aqueous sodium chloride. An obtained organic layer was dried by anhydrous sodium sulfate and was then condensed under reduced pressure, so that a crude product (1.83 g) of an isoquinuclidine derivative (17a) in which the methylene group adjacent to the nitrogen atom was converted into a carbonyl carbon was obtained in the form of a white solid material. The spectrum data of this isoquinuclidine derivative (17a) is as follows.

¹H NMR (CDCl₃):δ(ppm) 5.51 (dd, J = 5.3, 5.0 Hz, 1H), 5.05 (dd, J = 5.3, 0.9 Hz, 1H), 4.26 (dd, J = 3.9, 0.9 Hz, 1H), 3.19 (m, 1H), 2.88 (ddd, J = 10.6, 5.0, 0.7 Hz, 1H), 2.42 (ddd, J = 15.4, 10.6, 2.3 Hz, 1H), 2.25 (ddd, J = 15.4, 2.7, 0.7 Hz, 1H), 1.58 (s, 9H). :¹³C NMR (CDCl₃):δ(ppm) 174.1, 166.9, 150.1, 85.3, 79.9, 53.6, 47.5, 43.4, 34.8, 27.9, 26.2.

### Example 4

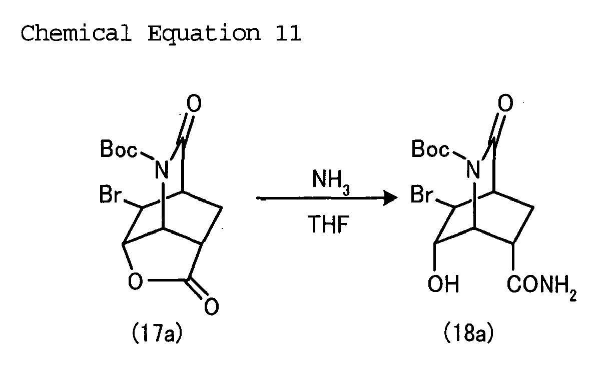

As shown in the chemical equation 11, after the isoquinuclidine derivative (17a) was dissolved in THF, an ammonium gas was introduced for 5 hours. After an argon gas was introduced into this reaction liquid, condensation thereof was performed under reduced pressure, so that a crude product (1.65 g) of an isoquinuclidine derivative (18a) having a hydroxyl group and an aminocarbonyl group was obtained in the form of a white crystal. The spectrum data of this isoquinuclidine derivative (18a) is as follows.

¹H NMR (CDCl₃):δ(ppm) 6.20-5.95 (brs, 1H), 5.85-5.60 (brs, 1H), 4.91 (m, 1H), 4.84 (dd, J = 3.2, 2.8 Hz, 1H), 4.39 (m, 1H), 4.08 (t, J = 3.2 Hz, 1H), 3.02 (m, 1H), 2.92 (ddd, J = 11.0, 6.9, 3.2 Hz, 1H), 2.37 (ddd, J = 14.2, 11.0, 4.2 Hz, 1H), 2.29 (ddd, J = 14.2, 6.9, 2.0 Hz, 1H), 1.56 (s, 9H). : ¹³C NMR (CDCl₃): δ(ppm) 175.6, 168.5, 149.8, 84.8, 79.0, 55.6, 50.6, 48.5, 41.8, 28.0, 27.5.

### Example 5

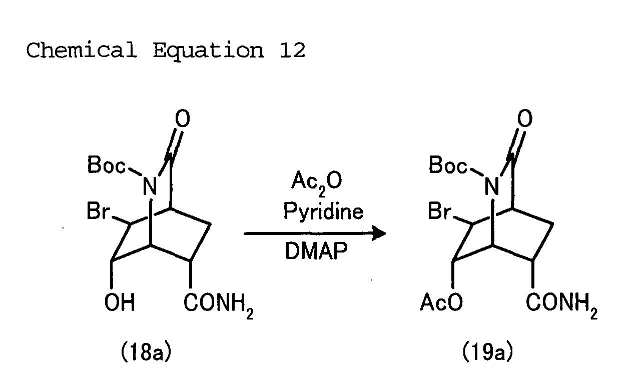

As shown in the chemical equation 12, after the isoquinuclidine derivative (18a) (1.61 g, 4.43 mmol) was dissolved in pyridine (30 ml), acetic anhydride (15 ml) and 4-dimethylaminopyridine (5.5 mg, 45 mmol) were added, and stirring was performed for 1 hour. This reaction liquid was condensed under reduced pressure, so that a yellow gummy material was obtained. The material was purified by column chromatography (80%-100% of ethyl acetate-hexane), so that an isoquinuclidine derivative (19a) in which the hydroxyl group was protected by an acetyl group was obtained in the form of a white crystal (1.45 g, 3.58 mmol, Y. 80.0% (3 stages)).

¹H NMR (CDCl₃):δ(ppm) 5.75-5.60 (brs, 1H), 5.50-5.30 (brs, 1H), 5.35 (dd, J = 3.4, 3.0 Hz, 1H), 5.18 (dd, J = 3.7, 3.2 Hz, 1H), 4.13 (dd, J = 3.0, 2.8 Hz, 1H), 3.05 (m, 1H), 2.85 (m, 1H), 2.65 (ddd, J = 14.9, 6.8, 2.1 Hz, 1H), 2.20 (ddd, J = 14.9, 10.8, 4.3 Hz, 1H), 2.07 (s, 3H), 1.60 (s, 9H).

### Example 6

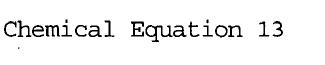

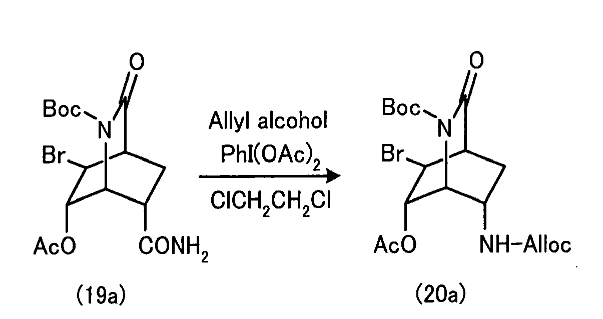

As shown in the chemical equation 13, after 1,2-dichloroethane (70 ml) and allyl alcohol (4.73 ml, 69.6 mmol) were added to the isoquinuclidine derivative (19a) (1.41 g, 3.48 mmol) and diacetoxyiodebenzene (3.35 g, 10.44 mmol) in an argon atmosphere, this mixture was heated to 80°C and was stirred for 2 hours. After cooling was performed to room temperature, and the reaction was stopped by adding a saturated sodium hydrogen carbonate aqueous solution and sodium thiosulfate were added, the reaction liquid was transferred to a separating funnel and was extracted twice by ethyl acetate. An organic layer was washed with saturated aqueous sodium chloride, was then dried by anhydrous sodium sulfate, and was further condensed under reduced pressure, so that a crude product (3.65 g) of an isoquinuclidine derivative (20a) having an allyl carbamate group was obtained. When this crude product was purified by column chromatography (ethyl acetate-20% to 50% of hexane), a pale yellow solid (1.20g, 2.60 mmol, Y. 74.7%) was obtained. The spectrum data of the isoquinuclidine derivative (20a) is as follows.

¹H NMR (CDCl₃):δ(ppm) 5.91 (m, 1H), 5.54 (s, 1H), 5.31 (d, J = 17.4 Hz 1H), 5.24 (d, J = 11.0 Hz, 1H), 5.15 (brd, J = 8.2 Hz, 1H), 4.81 (s, 1H), 4.58 (d, J = 5.5 Hz, 2H), 4.21 (m, 1H), 4.04 (s, 1H), 2.98 (brs, 1H), 2.74 (ddd, J = 14.7, 10.6, 3.7 Hz, 1H), 2.19 (s, 3H), 1.70-1.50 (m, 1H), 1.56(s, 9H).

### Example 7

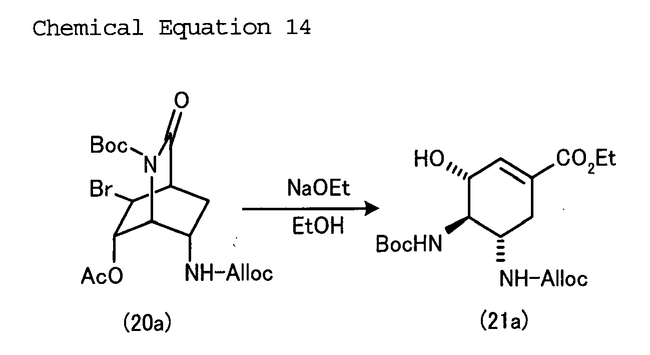

As shown in the chemical equation 14, after the isoquinuclidine derivative (20a) (360 mg, 0.780 mmol) was dissolved in ethanol (2 ml) and was then cooled to 0°C, sodium ethoxide (1 M ethanol solution, 1.56 ml, 1.56 mmol) was added. This mixture was stirred for 5 minutes at 0°C and was then further stirred for 30 minute at room temperature. This reaction liquid was diluted with dichloromethane, and the reaction was then stopped by adding diluted hydrochloric acid. The reaction liquid was extracted with ethyl acetate, and the total extracted liquid was dried by anhydrous sodium sulfate, followed by condensation. By purification using silica gel column chromatography (20% to 50% of ethyl acetate/hexane), a 1-cyclohexene-1-carboxylic acid derivative (21a) having a hydroxyl group at the 3-position was obtained in the form of a pale yellow foamed material (178 mg, Y. 59.4%). The spectrum data of this 1-cyclohexene-1-carboxylic acid derivative (21a) is as follows.

¹H NMR (CDCl₃):δ(ppm) 6.80 (s, 1H), 5.90 (m, 1H), 5.31 (br s, 1H), 5.30 (d, J = 17 Hz, 1H), 5.22 (d, J = 10.6 Hz, 1H), 5.18 (br s, 1H), 4.57 (m, 2H), 4.30 (m, 1H), 4.21 (q, J = 7.2 Hz, 2H), 3.82 (m, 1H), 3.57 (dd, J = 19.5, 8.0 Hz, 1H), 2.87 (dd, J = 17.4, 5.2 Hz, 1H), 2.23 (m, 1H), 1.44 (s, 9H), 1.29 (t, J = 7.2 Hz, 3H).

### Example 8

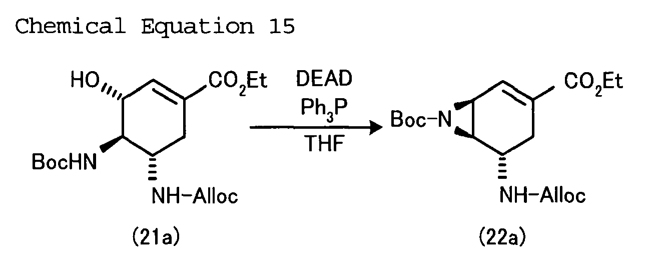

As shown in the chemical equation 15, in an argon atmosphere, triphenylphosphine (205 mg, 0.780 mmol) and tetrahydrofuran (7.2 ml) were supplied in a flame-dried test tube and was then cooled to 0°C. After diethyl azodicarboxylate (2.2 M toluene solution, 355 ml, 0.780 mmol) was further added, a tetrahydrofuran (3.6 ml) solution of the isoquinuclidine derivative (21a) (120 ml, 0.312 mmol) was added, and stirring was performed for 1 hour and 20 minutes. After triphenylphosphine (85 mg) and diethyl azodicarboxylate (2.2 M toluene solution, 140 ml) were added and were further stirred for 30 minutes, triphenylphosphine (87 mg) was added and was further stirred for 40 minutes, so that the Mitsunobu reaction was performed. The reaction liquid was condensed under reduced pressure and was then purified by column chromatography (ethyl acetate-20% to 22% of hexane), so that a 1-cyclohexene-1-carboxylic acid derivative (22a) (67.2 mg, 0.183 mmol, Y. 58.7%) having an aziridine was obtained. The spectrum data of this 1-cyclohexene-1-carboxylic acid derivative (22a) is as follows.

¹H NMR (CDCl₃):δ(ppm) 7.21 (t, J = 3.7 Hz, 1H), 5.90 (m, 1H), 5.30 (d, J = 17.4 Hz, 1H), 5.22 (d, J = 11.0 Hz, 1H), 4.61 (s, 2H), 4.56 (brd, J = 4.6 Hz, 1H), 4.20 (q, J = 7.3 Hz, 2H), 3.12 (d, J = 5.5 Hz, 1H), 2.99 (d, J = 5.5, 4.6 Hz, 1H), 2.73 (d, J = 17.4 Hz, 1H), 2.39 (d, J = 17.4 Hz, 1H), 1.62(m, 1H), 1.45 (s, 9H), 1.29 (t, J = 7.3 Hz, 3H).

### Example 9

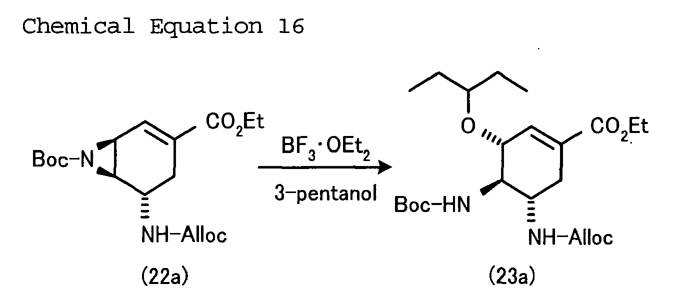

As shown in the chemical equation 16, after the 1-cyclohexene-1-carboxylic acid derivative (22a) (66.0 mg, 180 µmol) was dissolved in 3-pentanol (3.6 ml) and was then cooled to 0°C in an argon atmosphere, a trifluoroboron diethyl ether complex (3-pentanol solution, 0.1 M, 2.7 ml) was dripped for 15 minutes. After stirring was performed for 1 hour, the reaction was stopped by adding a saturated sodium hydrogen carbonate aqueous solution. This reaction liquid was extracted twice with ethyl acetate, and an obtained organic layer was washed with saturated aqueous sodium chloride, was then dried by anhydrous sodium sulfate, and was further condensed under reduced pressure, so that a crude product (83 mg) was obtained. This product was then purified by silica gel column chromatography (10% of ethyl acetate-hexane), so that a 1-cyclohexene-1-carboxylic acid derivative (23a) (37.4 mg, 82.3 µmol, Y. 45.7%) having a 1-ethylpropoxy group was obtained at the 3-position. The spectrum data of this 1-cyclohexene-1-carboxylic acid derivative (23a) is as follows.

¹H NMR (CDCl₃):δ(ppm) 6.80 (s, 1H), 5.89 (m, 1H), 5.64 (d, J = 8.2 Hz, 1H),
5.28 (brd, J = 16.5 Hz, 1H), 5.28 (d, J = 10.1 Hz, 1H), 4.65-4.45 (m, 1H), 4.54
(brs, 2H), 4.20 (q, J = 7.3 Hz, 2H), 3.94 (d, J = 5.5 Hz, 1H), 3.87 (m, 1H), 3.77 (m, 1H), 3.41 (quintet, J = 5.5 Hz, 1H), 2.75 (dd, J = 18.3, 8.3 Hz 1H), 2.34
(dd, J = 18.3, 8.3 Hz, 1H), 1.55 (dq, J = 7.3, 5.5 Hz, 4H), 1.42 (s, 9H), 1.28 (t, J = 7.3, 3H), 0.91 (t, J = 7.3 Hz, 6H).

### Example 10

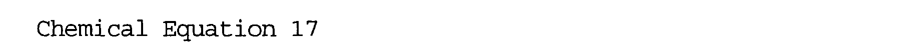

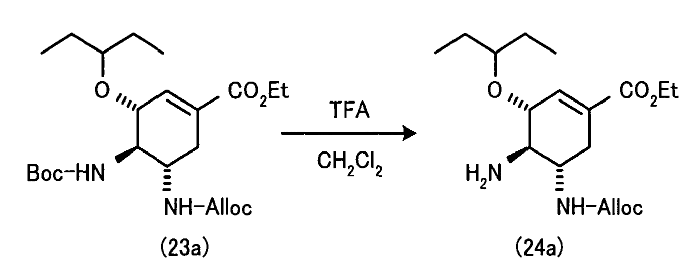

As shown in the chemical equation 17, in an argon atmosphere, the 1-cyclohexene-1-carboxylic acid derivative (23a) (37.4 mg, 82.3 µmol) was dissolved in dichloromethane (1.6 ml) and was then cooled to 0°C. Trifluoroacetic acid (244 µl, 3.29 mmol) was added, and stirring was then performed for 1 hour and 30 minutes. This reaction liquid was condensed under reduced pressure and was then purified by silica gel thin layer chromatography (ethyl acetate), so that a 1-cyclohexene-1-carboxylic acid derivative (24a) (30.9 mg) having an amino group at the 4-position was obtained. The spectrum data of this 1-cyclohexene-1-carboxylic acid derivative (24a) is as follows.

¹H NMR (CDCl₃):δ(ppm) 6.83 (s, 1H), 5.92 (m, 2H), 5.45-5.25 (brs, 1H), 5.31 (d, J = 17.2 Hz, 1H), 5.21 (d, J = 10.6 Hz, 1H), 4.57 (brd, J = 4.8 Hz, 1H), 4.21 (q, J = 7.1 Hz, 1H), 3.85 (m, 1H), 3.77 (m, 1H), 3.41 (quintet, J = 5.9 Hz, 1H), 2.91 (dd, J = 9.4, 6.9 Hz, 1H), 2.83 (dd J = 17.9, 4.8 Hz, 1H), 2.27 (dd, J = 17.9, 7.8 Hz, 1H), 1.57 (dq, J = 7.5, 5.9 Hz, 4H), 1.29 (t, J = 7.1 Hz, 3H), 0.93 (t, J = 7.5 Hz, 6H).

### Example 11

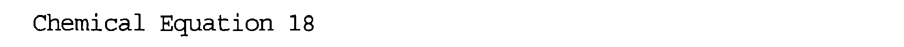

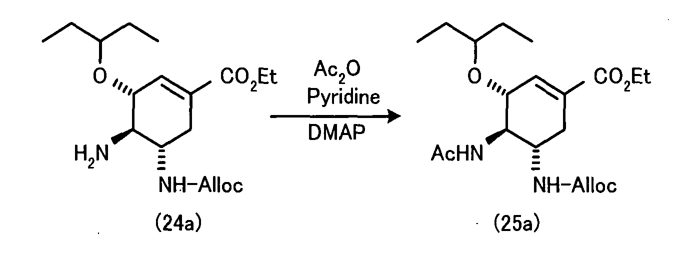

As shown in the chemical equation 18, the 1-cyclohexene-1-carboxylic acid derivative (24a) (30.9 mg, 87.2 µmol) was dissolved in pyridine, and anhydride acetic acid was added. Subsequently, after 4-dimethylaminopyridine (0.2 mg, 1.6 µmol) was added, stirring was performed for 1 hour, and this reaction liquid was condensed under reduced pressure. An obtained crude product was purified by silica gel thin layer chromatography (50% of ethyl acetate-hexane), so that a 1-cyclohexene-1-carboxylic acid derivative (25a) having an acetylamino group at the 4-position was obtained in the form of a white crystal (26.5 mg, 66.8 µmol, Y. 76.7%). The spectrum data of this 1-cyclohexene-1-carboxylic acid derivative (25a) is as follows.

¹H NMR (CDCl₃):δ(ppm) 6.81 (s, 1H), 5.89 (m, 1H), 5.62 (brd, J = 8.4 Hz, 1H), 5.55 (d, J = 9.3 Hz, 1H), 5.28 (d, J = 17.2 Hz, 1H), 5.20 (d, J = 10.5 Hz, 1H), 4.54 (m, 2H), 4.21 (q, J = 7.1 Hz, 2H), 4.11 (q, J = 9.0 Hz, 1H), 3.99 (m, 1H), 3.86 (m, 1H), 3.38 (quintet, J = 5.7 Hz, 1H), 2.77 (dd, J = 18.1, 4.8 Hz, 1H), 2.36 (dd, J = 18.1, 9.0 Hz, 1H), 1.98 (s, 3H), 1.52 (m, 4H), 1.29 (t, J = 7.1 Hz, 3H), 0.90 (dt, J = 8.8, 7.2 Hz, 6H). Example 12

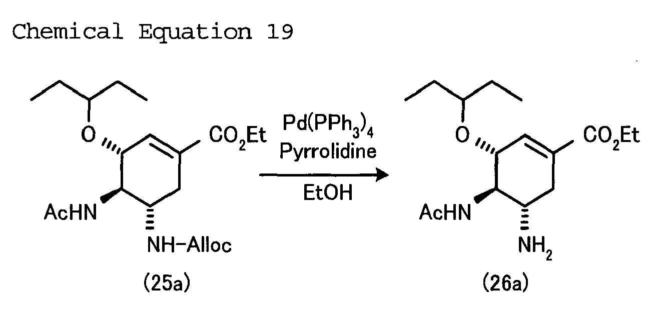

As shown in the chemical equation 19, after the 1-cyclohexene-1-carboxylic acid derivative (25a) (25.2 mg, 63.6 µmol) and tetrakis(triphenylphosphine)palladium (16.8 mg, 14.5 µmol) were dissolved in ethanol (1 ml) in an argon atmosphere, pyrrolidine (12 µl, 140 µmol) was added, and stirring was then performed for 30 minutes. After this reaction liquid was condensed under reduced pressure, was then dissolved in ethyl acetate, and was further extracted twice with hydrochloric acid (1 mol/l), an obtained aqueous layer was saturated by sodium bicarbonate and was then extracted three times with dichloromethane. An obtained organic layer was washed with saturated aqueous sodium chloride. The aqueous layer was extracted with dichloromethane, and a collected organic layer was dried by sodium sulfate and was then condensed under reduced pressure, so that a 1-cyclohexene-1-carboxylic acid derivative (26a) (18.9 mg, 60.5 µmol, Y. 95.1%) having a free amino group at the 5-position was obtained. The spectrum data of this 1-cyclohexene-1-carboxylic acid derivative (26a) is as follows.

¹H NMR (CDCl₃):δ(ppm) 6.79 (s, 1H), 5.70 (brd, J = 7.3 Hz, 1H), 4.21 (q, J = 7.3 Hz, 2H), 4.20(m, 1H), 3.55 (m, 1H), 3.34 (quintet, J = 6.0 Hz, 1H), 3.24 (m, 1H), 2.76 (dd, J = 17.4, 4.6 Hz, 1H), 2.16 (m, 1H), 2.05 (s, 3H), 1.51 (m, 4H), 1.29 (t, J = 7.3Hz, 3H), 0.90(dt, J = 7.3, 4.6 Hz, 6H).

### Example 13

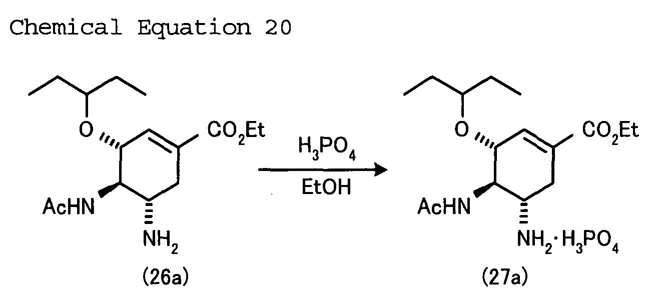

As shown in the chemical equation 20, after the 1-cyclohexene-1-carboxylic acid derivative (26a) (18.9 mg, 60.5 µmol) was dissolved in ethanol (450 ml), phosphoric acid (ethanol 1 M solution, 60.5 ml) was added, and condensation was performed under reduced pressure, so that a crystal was obtained. This crystal was washed with ethyl acetate and was then recrystallized from ethanol. The obtained crystal was washed twice with acetone, so that a phosphate (27a) (3.3 mg, 8.0 µmol, Y. 7.6%) was obtained in the form of a white crystal. The spectrum data of this phosphate (27a) is as shown below and coincided with the spectrum data of an authentic sample of oseltamivir. In addition, the spectrum data of the compounds (25a) and (26a) coincided with that obtained from a compound derived from the authentic sample of oseltamivir. Furthermore, since the optical rotation of the compound (25a) coincided with that of a compound having an allyl carbamate group derived from the amino group at the 5-position of the authentic sample of oseltamivir, the absolute configuration represented by each chemical formula was confirmed.

¹H NMR (D₂O):δ(ppm) 6.91 (s, 1H), 4.38 (brd, J = 8.3 Hz, 1H), 4.31 (m, 2H), 4.11 (dd, J = 11.9, 9.5 Hz, 1H), 3.62 (m, 2H),3.02(dd, J = 17.2, 5.8 Hz, 1H), 2.58 (m, 1H), 2.14 (s, 3H), 1.70-1.45 (m, 4H), 1.34 (t, J = 7.0 Hz, 3H), 0.94 (t, J = 7.8 Hz, 3H), 0.90 (t, J = 7.8 Hz, 3H).

In addition, as shown in chemical equation 21, when the isoquinuclidine derivative (17a) having a lactone was hydrolyzed by an acid and was then heated in the presence of anhydrous acetic acid and sodium acetate to form a cyclohexane dicarboxylic acid derivative having an oxazine, followed by decomposition thereof by ammonium, a 1-cyclohexene-1-carboxylic acid derivative having an acetoxy group at the 3-position, an acetylamino group at the 4-position, and an aminocarbonyl group at the 5-position may be formed.

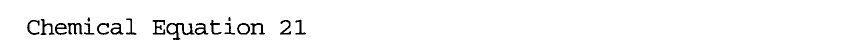

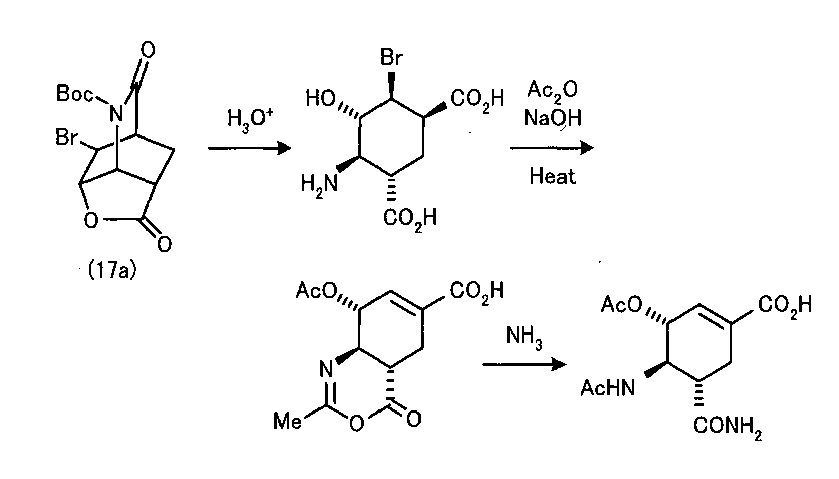

### Example 14

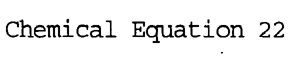

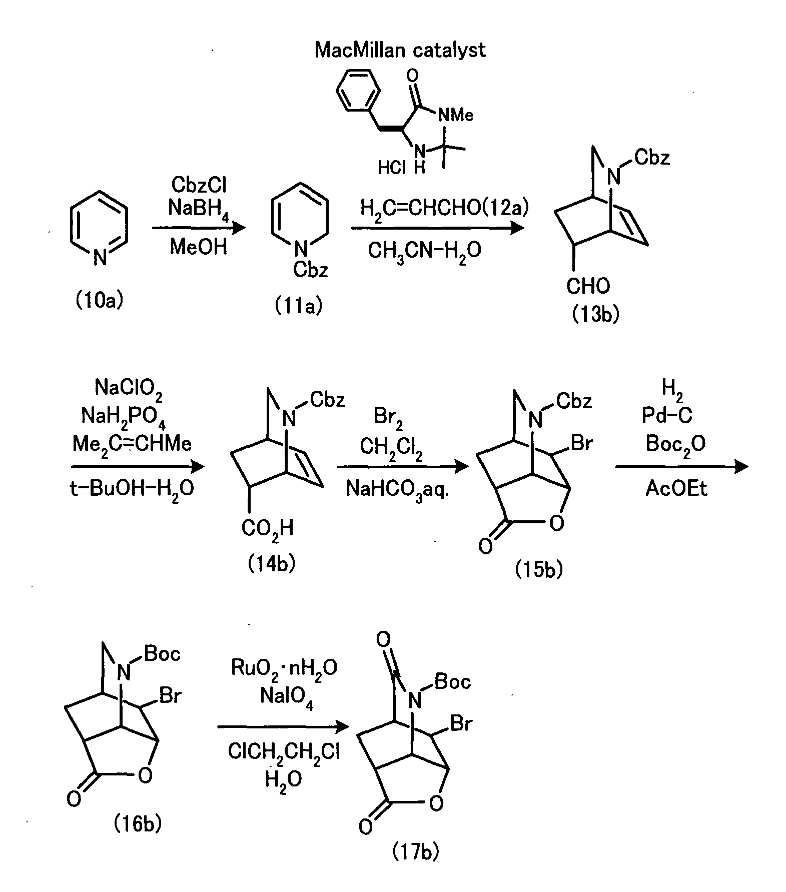

As shown in the chemical equation 22, reactions were performed in accordance with Examples 1 to 3 except that the Diels-Alder reaction was performed using the 5S configuration derived from L- phenylalanine as the MacMillan catalyst, so that an isoquinuclidine derivative (17b) was obtained in the form of a white crystal. This isoquinuclidine derivative (17b) was an enantiomeric isomer of the isoquinuclidine derivative (17a) described above, and the spectrum data of the isoquinuclidine derivative (17b) is as follows.

¹H NMR (CDCl₃): δ(ppm) 5.51 (dd, J = 5.5, 4.6 Hz, 1H), 5.05 (d, J = 5.5 Hz, 1H), 4.26 (d, J = 3.6 Hz, 1H), 3.19 (dt, J = 3.6, 1.4 Hz, 1H), 2.88 (dd, J = 10.1, 4.6 Hz, 1H), 2.42 (ddd, J = 14.7, 10.1, 1.4 Hz, 1H), 2.25 (dd, J = 14.7, 1.4 Hz, 1H), 1.58 (s, 9H).

### Example 15

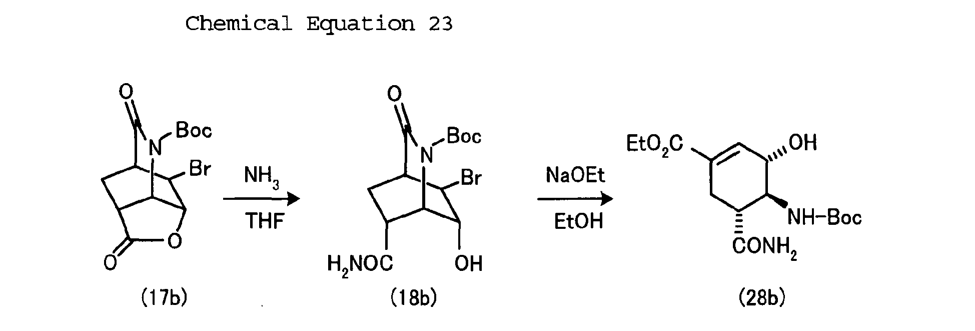

As shown in the chemical equation 23, the lactone ring of the isoquinuclidine derivative (17b) was opened in accordance with Example 4, so that an isoquinuclidine derivative (18b) having a hydroxyl group and an aminocarbonyl group was obtained. The spectrum data thereof is as follows.

¹H NMR (CDCl₃) :δ(ppm) 5.95(brs, 1H), 5.66 (brs, 1H), 4.89 (ddd, J = 10.1, 3.7, 2.8 Hz, 1H), 4.85 (t, J = 2.8 Hz, 1H), 4.39 (m, 1H), 4.08 (t, J = 2.8 Hz, 1H), 3.02 (dd, J = 1.8, 2.8 Hz, 1H), 2.91 (ddd, J = 8.2, 3.7, 2.8 Hz, 1H), 2.38 (ddd, J = 11.0, 4.6, 3.7 Hz, 1H), 2.29 (ddd, J = 14.7, 4.6, 3.7 Hz, 1H), 1.56 (s, 9H).

Subsequently, after ethanol (0.2 ml) was added to the isoquinuclidine derivative (18b) (7.0 mg, 20 µmol) in an argon atmosphere, sodium hydride (1.5 mg, 38 µmol) was added. After stirring was performed for 13 minutes, the reaction was stopped by adding acetic acid, and condensation was then performed under reduced pressure. After the condensed product was diluted with methylene chloride, saturated aqueous sodium chloride, saturated sodium bicarbonate water, and sodium chloride were sequentially added, and an organic layer was separated. The organic layer was dried using anhydrous sodium sulfate and was then condensed by under reduced pressure, so that a crude product (5.5 mg) was obtained. By purifying this product using preparative thin layer chromatography (7.5% of methanol-methylene chloride), a 1-cyclohexene-1-carboxylic acid derivative (28b) (2.2 mg, 7.1 µmol, 36%) was obtained. The spectrum data of this carboxylic acid derivative (28b) is as follows.

¹H NMR (CDCl₃):δ(ppm) 6.82 (s, 1H), 5.99 (brs, 1H), 5.50 (brs, 1H), 5.10 (d, J = 6.4 Hz, 1H), 4.53 (m, 1H), 4.21 (t, J = 7.3 Hz, 1H), 3.52 (m, 1H), 3.01 (m, 1H), 2.73 (dd, J = 5.5, 18.4 Hz, 1H), 2.62 (m, 1H), 1.44(s, 1H), 1.29 (t, J = 7.3 Hz, 3H).

### Example 16

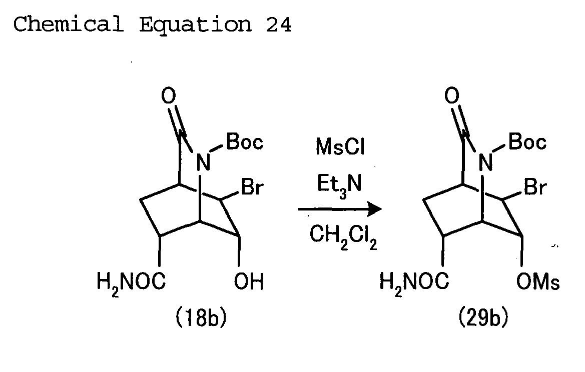

As shown in the chemical equation 24, the isoquinuclidine derivative (18b) (87.6 mg, 241 µmol) was dissolved in methylene chloride (2.5 ml) in an argon atmosphere, and triethylamine (106 µl, 759 µmol) and methanesulfonyl chloride (25.7 µl, 359 µmol) were then added, followed by stirring for 12 minutes. After this reaction liquid was diluted with methylene chloride and was then transferred to a separating funnel, a saturated sodium hydrogen carbonate aqueous solution was added, and the reaction liquid was saturated with sodium chloride. Subsequently, a small amount of ethanol was added, and an organic layer was then separated. An aqueous layer was extracted 7 times with methylene chloride, and after an obtained organic layer was dried by anhydrous sodium sulfate, condensation was performed under reduced pressure, so that a crude product (122 mg) was obtained. The crude product thus obtained was purified by silica gel thin layer chromatography (7.5% of methanol-methylene chloride), so that an isoquinuclidine derivative (29b) having a mesyloxy group and an aminocarbonyl group was obtained in the form of a white crystal (84.1 mg, 76.5%). The spectrum data of this isoquinuclidine derivative (29b) is as follows.

¹H NMR (CDCl₃):δ(ppm) 5.82 (brs, 1H), 5.58 (brs, 1H), 5.21 (dd, J = 3.7, 2.8 Hz, 1H), 5.14 (dd, J = 3.7, 3.6 Hz, 1H), 4.25 (dd, J = 3.6, 2.8 Hz, 1H), 3.13 (s, 3H), 3.04 (dd, J = 3.7, 2.8 Hz, 1H), 2.95 (m, 1H), 2.76 (ddd, J = 14.7, 6.4, 2.8Hz, 1H), 2.17 (ddd, J = 14.7, 11.0, 3.7 Hz, 1H), 1.61 (s, 9H). Example 17

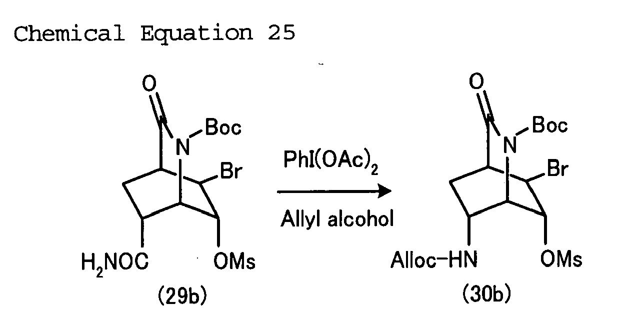

As shown in the chemical equation 25, after 1,2-dichloroethane (0.8 ml) and allyl alcohol (55 µl, 796 µmol) were added to the isoquinuclidine derivative (29b) (17.5 mg, 39.8 µmol) and diacetoxyiodebenzene (39.3 mg, 119 µmol) in an argon atmosphere, this mixture was heated to 80°C and was stirred fro 1 hour and 30 minutes. Next, after the mixture was cooled to room temperature and was diluted with methylene chloride, it was transferred to a separating funnel, and a saturated sodium hydrogen carbonate aqueous solution and sodium thiosulfate pentahydrate were added. After an organic layer was separated, was then washed saturated aqueous sodium chloride; and was further dried by anhydrous sodium sulfate, condensation was performed under reduced pressure, so that a crude product (26.7 mg) was obtained. The crude product thus obtained was purified by silica gel thin layer chromatography (methylene chloride), so that an isoquinuclidine derivative (30b) having a mesyloxy group and an allyl carbamate group was obtained in the form of a colorless oily material (17.1 mg, 86%). The spectrum data of this isoquinuclidine derivative (30b) is as follows.

¹H NMR (CDCl₃):δ(ppm) 5.91 (m, 1H), 5.34-5.22 (m, 2H), 5.29-5.22 (m, 1H), 4.97 (s, 1H), 4.59 (d, J = 5.5 Hz, 2H), 4,25 (m, 1H), 4.22 (dd, J = 2.8, 1.4 Hz, 1H), 3.19 (s, 1H), 3.00 (m, 1H), 2.76 (ddd, J = 14.6, 11.0, 4.6 Hz, 1H), 1.64 (ddd, 14.6, 6.4, 1.8 Hz), 1.56(s, 9H).

### Example 18

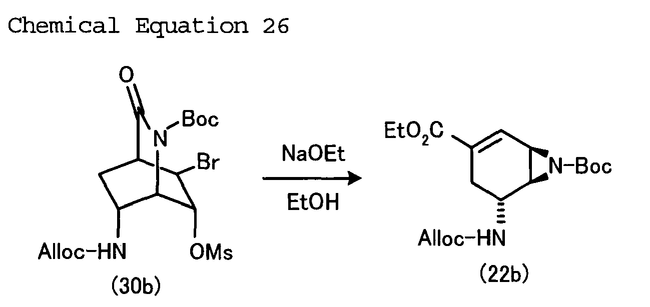

As shown in the chemical equation 26, after ethanol (0.1 ml) was added to the isoquinuclidine derivative (30b) (4.7 mg, 9.5 µmol) in an argon atmosphere, sodium ethoxide (ethanol solution, 0.2 M, 90.5 µl) was dripped for 6 minutes. After stirring was performed for 1 minute, this reaction liquid was diluted with ethyl acetate, and saturated sodium salt water, 1 M hydrochloric acid, and sodium chloride were sequentially added, so that an organic layer was separated. After an aqueous layer was extracted with ethyl acetate, obtained organic layers were collectively dried by anhydrous sodium sulfate and were then condensed under reduced pressure, so that a crude product (3.0 mg) was obtained. The crude product was purified by silica gel thin layer chromatography (20% of ethyl acetate-hexane), and a 1-cyclohexene-1-carboxylic acid derivative (22b) having an aziridine was obtained in the form of an oily material (2.6 mg, 7.1 µmol). This carboxylic acid derivative (22b) is an enantiomeric isomer of the above-described carboxylic acid derivative (22a), and the spectrum data of the carboxylic acid derivative (22b) is as follows.

¹H NMR (CDCl₃):δ(ppm) 5.51 (dd, J = 5.5, 4.6 Hz, 1H), 5.05 (d, J = 5.5 Hz, 1H), 4.26 (d, J = 3.6 Hz, 1H), 3.19 (dt, J = 3.6, 1.4 Hz, 1H), 2.88 (dd, J = 10.1, 4.6 Hz, 1H), 2.42 (ddd, J = 14.7, 10.1, 1.4 Hz, 1H), 2.25 (dd, J = 14.7, 1.4 Hz, 1H), 1.58 (s, 9H).

### Example 19

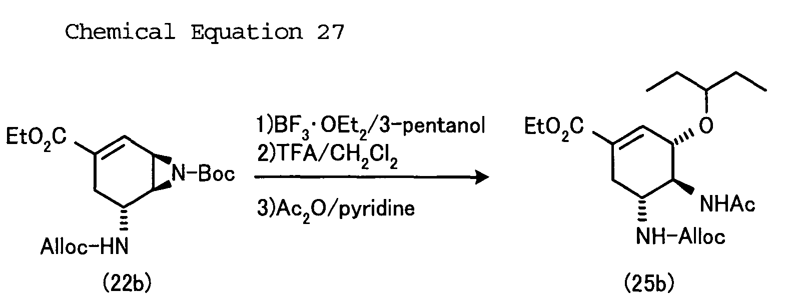

As shown in the chemical equation 27, in accordance with Examples 9 to 11, a 1-ethylpropoxy group was introduced to the 3-position of the 1-cyclohexene-1-carboxylic acid derivative (22b) having an aziridine, and after a free amino group was obtained by removing the Boc group used as a protective group therefor, the free amino group was protected by an acetyl group, so that a 1-cyclohexene-1-carboxylic acid derivative (25b) having a 1-ethylpropoxy group at the 3 position, an acetylamino group at the 4 position, and an allyloxycarbonylamino group at the 5 position was obtained in the form of an oily material. The proton NMR of this carboxylic acid derivative (25b) coincided
with that of a compound derived from the authentic sample of oseltamivir.

### Example 20

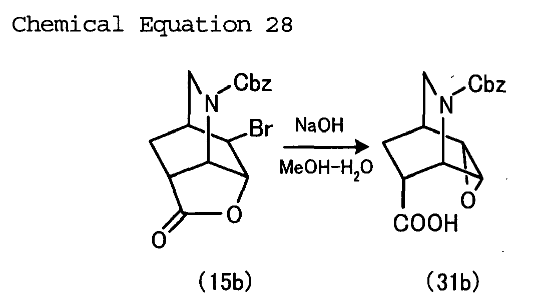

As shown in the chemical equation 28, after an isoquinuclidine derivative (15b) (14 mg, 38 µmol) was dissolved in methanol, 1 M aqueous solution of sodium hydroxide (0.2 ml, 153 µmol) was added, and stirring was performed at 50°C for 4 hours. Subsequently, the reaction was stopped by hydrochloric acid, and extraction was performed with ethyl acetate. A collected organic layer was dried by anhydrous sodium sulfate and was condensed, so that an isoquinuclidine derivative (31b) (11 mg, Y. 95%) having an epoxy was obtained. The spectrum data of this isoquinuclidine derivative (31b) is as follows.

¹H NMR (CDCl₃):δ(ppm) 7.37 (m, 4H), 5.16 (s, 2H), 5.07 (t, J = 4.1 Hz, 1/2(1H)), 4.96 (t, J = 3.7 Hz, 1/2(1H)), 3.55 (t, J = 4.6 Hz, 1/2(1H)), 3.50 (t, J = 4.6 Hz, 1/2(1H)), 3.40-3.32 (m, 3H), 2.72 (m, 1H), 2.49 (m, 1H), 2.29 (m, 1H), 1.68 (m, 1H).

In addition, by a synthesis route shown in chemical equation 29, a 1-cyclohexene-1-carboxylic acid derivative (35b) having a hydroxyl group at the 3 position, a benzyloxycarbonylamino group at the 4 position, and an amino group at the 5 position which is protected by an allyloxycarbonyl group can be obtained from the isoquinuclidine derivative (31b) through an isoquinuclidine derivative (32b) having an amino group protected by a Boc group, an isoquinuclidine derivative (33b) in which a carbon adjacent to the N atom is oxidized into a carbonyl carbon, and an isoquinuclidine derivative (34b) in which the carboxyl group is converted into a substituted amino group.

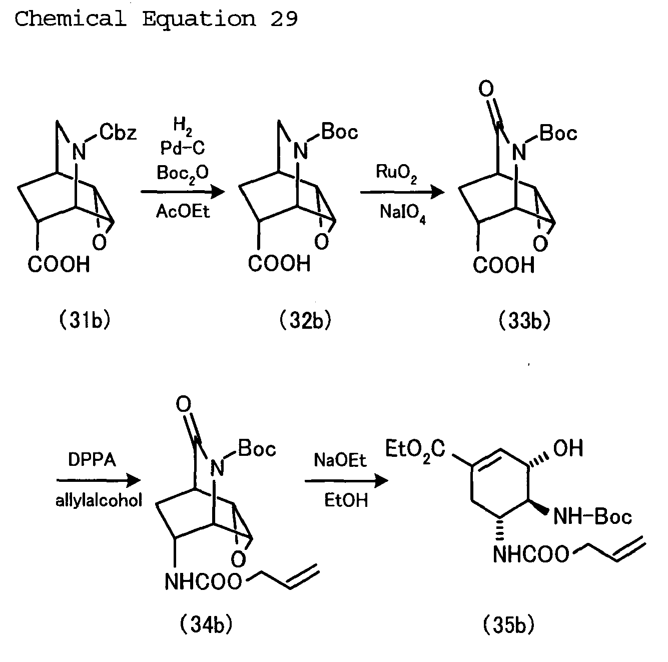

### Example 21

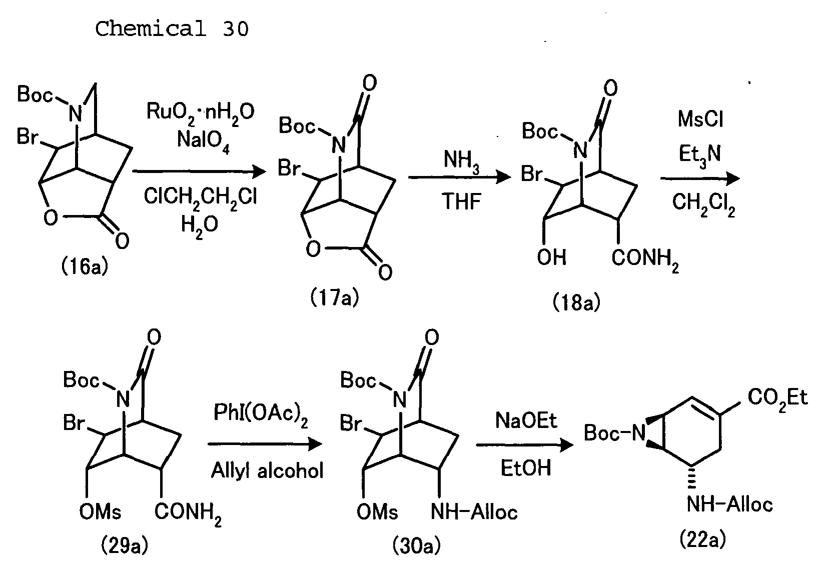

As shown in the chemical equation, the 1-cyclohexene-1-carboxylic acid derivative (22a) having an aziridine was synthesized from the isoquinuclidine derivative (16a) obtained in Example 2 through five reaction stages. Hereinafter, the details will be described.

After 1,2-dichloroethane (8.0 ml) and water (4.0 ml) were added to the isoquinuclidine derivative (16a) (507 mg, 1.53 mmol) and ruthenium dioxide n-hydrate (37.8 mg, 0.153 mmol) in an argon atmosphere, sodium periodate (1.31 g, 6.12 mmol) was also added, and this mixture was heated to 80°C, and was stirred for 2 hours and 40 minutes. Next, after cooling was performed to 0°C, the reaction was stopped by adding 30 drops of 2-propanol. After a precipitate was processed by celite filtration, the filtrate was diluted with ethyl acetate, was transferred to a separating funnel, and was then washed with a saturated sodium hydrogen carbonate aqueous solution. After an aqueous layer was extracted with ethyl acetate, a collected organic layer was washed with saturated aqueous sodium chloride. The organic layer thus obtained was dried by anhydrous sodium sulfate and was then condensed under reduced pressure, so that a crude product of the isoquinuclidine derivative (17a) in which the methylene group adjacent to the nitrogen atom was converted into a carbonyl carbon was obtained in the form of a white solid material. Subsequently, this isoquinuclidine derivative (17a) was dissolved in THF, and an ammonium gas was introduced thereinto for 3 hours and 30 minutes. After an argon gas was introduced into this reaction liquid, condensation was performed under reduced pressure, so that a crude product of the isoquinuclidine derivative (18a) having a hydroxyl group and an aminocarbonyl group was obtained in the form of a white crystal. Next, this isoquinuclidine derivative (18a) was dissolved in methylene chloride (16 ml) in an argon atmosphere, and triethylamine (0.700 ml, 5.02 mmol) and methanesulfonyl chloride (144 ml, 1.86 mmol) were added, followed by stirring for 12 minutes. After this reaction liquid was diluted with methylene chloride and was then transferred to a separating funnel, a saturated sodium hydrogen carbonate aqueous solution was added, the reaction liquid was saturated with sodium chloride, and an organic layer was separated. After an aqueous layer was extracted twice with methylene chloride, and an obtained organic layer was dried by anhydrous sodium sulfate, a crude product of an isoquinuclidine derivative (29a) having a mesyloxy group and an aminocarbonyl group was obtained by performing condensation under reduced pressure. The obtained crude product was purified by column chromatography (3% to 100 of methanol-methylene chloride), so that the isoquinuclidine derivative (29a) was obtained in the form of a white crystal (589 mg, 1.34 mmol, 87.6% through 3 reaction stages).

Subsequently, 1,2-dichloroethane (27 ml), diacetoxyiodebenzene (1.33 mg, 4.03 mmol), and allyl alcohol (1.83 mmol, 26.8 mmol) were sequentially added in an argon atmosphere to the isoquinuclidine derivative (29a) (588 mg, 1.34 mmol) thus obtained, and this mixture was heated to 80°C and was stirred for 1 hour and 30 minutes. Next, after the reaction liquid was cooled to room temperature, was diluted with methylene chloride, and was then transferred to a separating funnel, a saturated sodium hydrogen carbonate aqueous solution and a saturated sodium thiosulfate aqueous solution were added. An organic layer was separated, and an aqueous layer was extracted with methylene chloride. After a collected organic layer was washed with saturated aqueous sodium chloride, and an aqueous layer was then extracted with methylene chloride, a collected organic layer was dried by anhydrous sodium sulfate. By condensation under reduced pressure, a crude product (1.33 g) of an isoquinuclidine derivative (30a) having a mesyloxy group and an allyl carbamate group was obtained. When this crude product was purified by column chromatography (50% of ethyl acetate-hexane), this isoquinuclidine derivative (30a) was obtained in the form of a foamed white solid material (452 mg, 0.909 mmol, 67.8%).

After ethanol (9.0 ml) was added in an argon atmosphere to the isoquinuclidine derivative (30a) (452 mg, 0.909 mmol) thus obtained, this mixture was cooled to 0°C, and sodium ethoxide (ethanol solution, 1.0 M, 1.82 ml) was dripped for 3 minutes. After this mixture was stirred for 10 minutes, sodium ethoxide (ethanol solution, 1.0 M, 0.3 ml) was added thereto, and stirring was performed for 6 minutes. Next, after sodium ethoxide (ethanol solution, 1.0 M, 0.2 ml) was added, and stirring was performed for 6 minutes, sodium ethoxide (ethanol solution, 1.0 M, 0.2 ml) was further added, and stirring was performed for 7 minutes. Subsequently, after sodium ethoxide (ethanol solution, 1.0 M, 0.16 ml) was added, and stirring was performed for 6 minutes, this reaction liquid was diluted with methylene chloride and was then washed with saturated sodium chloride. An aqueous layer was extracted twice with methylene chloride, and obtained organic layers were then collectively dried by anhydrous sodium sulfate, so that a crude product (382 mg) was obtained by condensation under reduced pressure. This crude product was purified by column chromatography (33% of ethyl acetate-hexane), so that the 1-cyclohexene-1-carboxylic acid derivative (22a) having an aziridine in an amount of 277 mg (0.757 mmol, 83.3%) was obtained. In Example 18, hydrochloric acid was used in a post-treatment; however, since the aziridine ring was opened in some cases when hydrochloric acid was used, and the reproducibility was not always stable thereby, hydrochloric acid was not used in this example. As a result, the reproducibility and the yield were both improved as compared to those in Example 18. Subsequently, in accordance with Example 19, the 1-cyclohexene-1-carboxylic acid derivative (22a) having an aziridine can be formed into the oseltamivir (25a) (see the chemical equation 18).

### Industrial Applicability

The present invention can be used in the drug field, in particular, in the medicine field. In particular, the present invention can be used for synthesis of a 1-cyclohexene-1-carboxylic acid derivative (such as oseltamivir).

## Claims

1. An isoquinuclidine derivative represented by formula (1) or an enantiomer thereof: 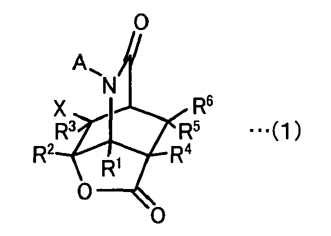 where in the formula (1), A represents a protective group for a nitrogen atom; R¹ to R⁶ each independently represent an alkyl group which may have a substituent, an aryl group which may have a substituent, or a hydrogen atom; and X represents a halogen atom.

2. An isoquinuclidine derivative represented by formula (2) or an enantiomer thereof: 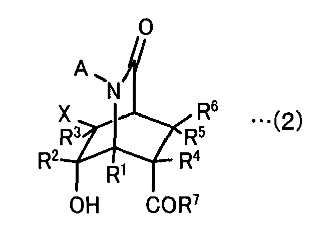 where in the formula (2), A represents a protective group for a nitrogen atom; R¹ to R⁶ each independently represent an alkyl group which may have a substituent, an aryl group which may have a substituent, or a hydrogen atom; R⁷ represents a hydroxyl group, an amino group which may have a substituent, a hydrazino group which may have a substituent, an alkoxy group which may have a substituent, or a hydroxyamino group which may have a substituent; and X represents a halogen atom.

3. An isoquinuclidine derivative represented by formula (3) or an enantiomer thereof: 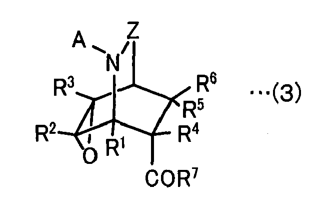 where in the formula (3), A represents a protective group for a nitrogen atom; R¹ to R⁶ each independently represent an alkyl group which may have a substituent, an aryl group which may have a substituent, or a hydrogen atom; R⁷ represents a hydroxyl group, an amino group which may have a substituent, a hydrazino group which may have a substituent, an alkoxy group which may have a substituent, or a hydroxyamino group which may have a substituent; and Z represents a -CH₂- group or a -C(=O)- group.

4. The isoquinuclidine derivative according to one of Claims 1 to 3, wherein the isoquinuclidine derivative is used as a synthetic intermediate for manufacturing a 1-cyclohexene-1-carboxylic acid derivative.

5. The isoquinuclidine derivative according to Claim 4, wherein the 1-cyclohexene-1-carboxylic acid derivative includes oseltamivir.

6. A method for manufacturing a 1-cyclohexene-1-carboxylic acid derivative comprising:
a step (a1) of, after the -OH group of the isoquinuclidine derivative according to Claim 2 is protected or is converted into an elimination group, converting the -COR⁷ group into a non-substituted or a substituted amino group by a rearrangement reaction; and
a step (b1) of disconnecting the bond between a nitrogen atom and a carbonyl carbon forming a lactam of an isoquinuclidine derivative obtained in the step (a1) and eliminating a hydrogen atom at the α position and X at the β position of the carbonyl carbon forming the lactam to form an unsaturated bond.

7. A method for manufacturing a 1-cyclohexene-1-carboxylic acid derivative comprising:
a step of disconnecting the bond between the nitrogen atom and the carbonyl carbon forming the lactam of the isoquinuclidine derivative according to Claim 2 and eliminating the hydrogen atom at the α position and X at the β position of the carbonyl carbon forming the lactam to form an unsaturated bond.

8. The method for manufacturing a 1-cyclohexene-1-carboxylic acid derivative according to Claim 7,
wherein in the step, after the -OH group of the isoquinuclidine derivative according to Claim 2 is protected or is converted into an elimination group, the bond between the nitrogen atom and the carbonyl carbon forming the lactam of the isoquinuclidine derivative is disconnected.

9. The method for manufacturing a 1-cyclohexene-1-carboxylic acid derivative according to one of Claims 6 to 8,
wherein as the isoquinuclidine derivative according to Claim 2, a compound is used which is obtained by disconnecting the bond between the oxygen atom and the carbonyl carbon forming the lactone of the isoquinuclidine derivative according to Claim 1.

10. A method for manufacturing a 1-cyclohexene-1-carboxylic acid derivative comprising:
a step (a2) of converting the -COR⁷ group of the isoquinuclidine derivative (where Z represents a -C(=O)- group) according to Claim 3 into a non-substituted or a substituted amino group by a rearrangement reaction; and
a step (b2) of disconnecting the bond between a nitrogen atom and a carbonyl carbon forming a lactam of an isoquinuclidine derivative obtained in the step (a2) and forming an unsaturated bond between the α position and the β position of the carbonyl carbon forming the lactam concomitant with opening of an epoxy ring.

11. A method for manufacturing a 1-cyclohexene-1-carboxylic acid derivative comprising:
a step of disconnecting the bond between the nitrogen atom and the carbonyl carbon forming the lactam of the isoquinuclidine derivative (where Z represents a -C(=O)- group) according to Claim 3 and forming an unsaturated bond between the α position and the β position of the carbonyl carbon forming the lactam concomitant with opening of the epoxy ring.

12. The method for manufacturing a 1-cyclohexene-1-carboxylic acid derivative according to one of Claims 6 to 11, wherein the 1-cyclohexene-1-carboxylic acid derivative includes oseltamivir.
